(19) Europäisches Patentamt
European Patent Office
Office européen des brevets

(11) **EP 4 463 084 B1**

(12) **EUROPEAN PATENT SPECIFICATION**

(45) Date of publication and mention
of the grant of the patent:
**20.05.2026   Bulletin 2026/21**

(21) Application number: **23709888.4**

(22) Date of filing: **07.02.2023**

(51) International Patent Classification (IPC):
*A61B 17/29* (2006.01)      *A61B 17/072* (2006.01)
*A61B 34/30* (2016.01)

(52) Cooperative Patent Classification (CPC):
**A61B 17/29; A61B 17/07207;** A61B 34/71;
A61B 2017/0034; A61B 2017/00398;
A61B 2017/07214; A61B 2017/2903;
A61B 2017/2933; A61B 2017/2937;
A61B 2017/2939; A61B 2017/2943

(86) International application number:
**PCT/US2023/062090**

(87) International publication number:
**WO 2023/154689 (17.08.2023 Gazette 2023/33)**

(54) **MEDICAL DEVICES AND INSTRUMENTS**

MEDIZINISCHE VORRICHTUNGEN UND INSTRUMENTE

DISPOSITIFS MÉDICAUX ET INSTRUMENTS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **08.02.2022   US 202263267702 P**

(43) Date of publication of application:
**20.11.2024   Bulletin 2024/47**

(73) Proprietor: **Boston Scientific Scimed Inc.
Maple Grove, Minnesota 55311-1566 (US)**

(72) Inventors:
• **FLANAGAN, Aiden
Kilcolgan, Co. Galway, H91FV1X (IE)**
• **MCCOOEY, Stephen
Louth Village, Dundalk (IE)**
• **FAWDRY, Martin, Lawrence
Claregalway, Co. Galway, H91K5Y4 (IE)**

(74) Representative: **Vossius & Partner
Patentanwälte Rechtsanwälte mbB
Siebertstrasse 3
81675 München (DE)**

(56) References cited:
DE-A1- 102012 222 755     US-A1- 2013 079 810
US-A1- 2015 066 022       US-A1- 2018 256 182
US-A1- 2019 125 357       US-A1- 2019 380 800
US-A1- 2020 305 870       US-B2- 9 095 362

Description

## TECHNICAL FIELD

Various aspects of the disclosure relate generally to medical devices for various procedures, such as, e.g., endoscopy, colonoscopy, etc. More specifically, at least certain embodiments of the disclosure relate to devices including instruments or tools (e.g., tissue-fasteners, forceps, etc.) that may be actuated via application of a rotational force, among other aspects.

## BACKGROUND

Medical devices may include an instrument or tool coupled or fixed to a distal end of a shaft, and a controller or an actuator positioned at or around a proximal end of the shaft. In order to actuate the instrument or tool at the distal end of the shaft, a sufficient degree of force must be applied in a controlled and directed manner. For example, in some devices, the distal instrument may be coupled to the actuator via one or more wires, and the pulling of the wire(s) via the actuator may impart a force actuating or operating the instrument. However, it is difficult to transfer a force applied externally from the proximal side of the shaft to actuate an instrument at the distal end of the shaft. For example, the force applied via a proximal actuator may be reduced due to the shaft being in tortuous anatomy, resulting in bending and constraint of the aforementioned pull wires of the shaft. Miniaturization of surgical instruments or tools may further reduce the efficiency and capability of distally mounted tools and also may reduce the room available for proximal-to-distal actuation mechanisms, such as pull wires.

US 2019/125357 A1 discloses a surgical device for clipping tissue, having a clip crimping system comprising a clamp operably coupled with an electric motor, where the clamp is moved distally by the electric motor to perform a crimping stroke and engage a first jaw and a second jaw.

## SUMMARY

The invention is defined by claim 1. Further embodiments of the invention are defined by the dependent claims. Surgical methods are not claimed.

According to an example, a medical device may comprise an end-effector, including a first feature and a second feature, wherein the first feature and the second feature are pivotably joined to one another, and a motor coupled to a rotatable shaft, wherein the rotatable shaft engages the end-effector, and wherein the motor is configured to rotate the rotatable shaft in a first direction and a second direction, wherein the second direction is opposite of the first direction, wherein a first rotation of the rotatable shaft in the first direction is configured pivot the first feature and the second feature towards one another, and wherein a second rotation of the rotatable shaft in the second direction is configured to pivot the first feature and the second feature away from one another.

In another example, the medical device may further comprise a connector coupled to the rotatable shaft, wherein the connector is configured to directly engage the end effector. The medical device may further comprise a first cable, including a first end coupled to the connector and a second end coupled to the first feature, and a second cable, including a first end coupled to the connector and a second end coupled to the second feature, wherein the connector includes a threaded lumen, wherein the rotatable shaft includes a threaded exterior, engaging the threaded lumen of the connector, and wherein the first rotation of the rotatable shaft in the first direction translates the connector in a first longitudinal direction, and wherein the second rotation of the rotatable shaft in the second direction translates the connector in a second longitudinal direction. A first translation of the connector in the first longitudinal direction may pivot the first aspect and the second aspect towards one another, and the second translation of the connector in the second longitudinal direction may cause the first cable to pull on the first feature and the second cable to pull on the second feature, thereby pivoting the first feature and the second feature away from another.

In another example, the connector may include a threaded lumen, wherein the end-effector further includes a body proximal to the first feature and the second feature, wherein the body includes a threaded exterior, wherein the threaded lumen of the connector engages the threaded exterior of the body of the end-effector, and wherein the first rotation of the rotatable shaft in the first direction translates the end-effector in a first longitudinal direction, relative to the connector, and wherein second rotation of the rotatable shaft in the second direction translates the end-effector in a second longitudinal direction, relative to the connector. The first feature may include a first threading and the second feature includes a second threading, wherein the threaded lumen of the connector engages the first threading of the first feature and the second threading of the second feature, and wherein a first translation of the end-effector in the first longitudinal direction pivots the first feature and the second feature towards one another, and wherein a second translation of the end-effector in the second longitudinal direction pivots the first feature and the second feature away from another. The medical device may further comprise a sheath, sheathing at least a portion of the connector and at least a portion of the end-effector, wherein the sheath includes a first longitudinal slot through which the first feature of the end-effector extends and retracts, and a second longitudinal slot through which the second feature of the end-effector extends and retracts, a first cable, including a first end coupled to the sheath and a second end coupled to the first feature, and a second cable, including a first end coupled to the sheath and a second end coupled to the second feature, wherein a first translation of the end-effector in the first longitudinal direction pivots the first aspect and the second aspect towards one another, and wherein a second translation of the end-effector in the second longitudinal direction pivots the first aspect and the second aspect away from another.

in another example, the connector may comprise a cylindrical rod, and wherein at least a portion of the cylindrical bar includes a threaded exterior, and wherein the end-effector engages the threaded exterior, such that (a) the first rotation of the shaft in the first direction causes the end-effector to longitudinally translate along the threaded exterior in a first longitudinal direction, and (b) the second rotation of the shaft in the second direction causes the end-effector to longitudinally translate along the threaded exterior in a second longitudinal direction.

In another example, the connector may include an intermediary gear and a threaded bar, wherein the threaded bar includes (a) a first threaded portion fixed to and extends from a center of a first side of the intermediary gear, and (b) a second threaded portion fixed to and extending from the center of a second side of the intermediary gear, wherein the rotatable shaft includes a threaded exterior engaging the intermediary gear, wherein the first feature of the end effector engages the first threaded portion of the threaded bar, and wherein the second feature of the end effector engages the second threaded portion of the threaded bar. The first threaded portion may be threaded in a first direction and the second threaded portion is threaded in a second direction, and wherein the second direction is opposite of the first direction.

In another example, the medical device may further comprise a first cable, including a first end coupled to the connector and a second end coupled to the first feature, and a second cable, including a first end coupled to the connector and a second end coupled to the second feature, wherein the connector includes a threaded lumen, wherein the rotatable shaft includes a threaded exterior, engaging the threaded lumen of the connector, and wherein the first rotation of the rotatable shaft in the first direction translates the connector in a first longitudinal direction, and wherein the second rotation of the rotatable shaft in the second direction translates the connector in a second longitudinal direction. The connector may include a first side coupled to the rotatable shaft, and a second side including a spiral thread formed thereon, wherein the first feature of the end-effector includes a first end engaging a groove of the spiral thread, and wherein the second feature of the end-effector includes a second end engaging the groove of the spiral thread.

In another example, the rotatable shaft may include a threaded exterior, and the second feature of the end-effector may include a proximal surface including a first plurality of teeth engaging the threaded exterior of the rotatable shaft. The first feature of the end-effector may include a proximal surface including a second plurality of teeth engaging the threaded exterior of the rotatable shaft, and the rotatable shaft may be positioned between the proximal surface of the first aspect and the proximal surface of the second aspect.

In another example, the end-effector may be a stapler, wherein the first feature includes an anvil of the stapler, and wherein the second feature includes a cartridge housing of the stapler.

In another example, the medical device may further comprise a controller in communication with the motor, wherein the controller is configured to select and/or change a direction of the rotation of the shaft imparted by the motor.

According to another example, a medical device may comprise a shaft, an end-effector, wherein the end-effector includes a first aspect and a second aspect, and a rotating body, wherein an exterior of the rotating body includes one or more of (a) teeth or (b) threads, and wherein the rotating body is configured to rotate in a first direction and a second direction, wherein the second direction is opposite to the first direction, wherein a first rotation of the rotatable shaft in the first direction is configured move the first feature and the second feature towards one another, and wherein a second rotation of the rotatable shaft in the second direction is configured to move the first feature and the second feature away from one another. The actuation mechanism may further include a motor configured to rotate the body in the first direction and the second direction. The first feature may include a first leg and a first arm, wherein the first arm is perpendicular to the first leg, wherein the second aspect includes a second leg and a second arm, and wherein the second arm is perpendicular to the second leg. The first leg may include a first plurality of teeth engaging the body, wherein the second leg includes a second plurality of teeth engaging the gear, and wherein the first side and the second side are approximately parallel.

According to an example, a method of treatment may comprise inserting a medical instrument into a subject, wherein the medical instrument includes a first feature and a second feature, wherein at least the first feature is pivotable relative to the second feature, delivering the medical instrument to a target tissue, activating a motor of the medical instrument, thereby rotating a rotatable shaft of the medical instrument, wherein a rotation of the rotatable shaft engages the first feature, thereby pivoting the first feature relative to the second feature.

## BRIEF DESCRIPTION OF THE DRAWINGS

The accompanying drawings, which are incorporated in and constitute a part of this specification, illustrate exemplary aspects of the disclosure and together with the description, serve to explain the principles of the disclosure.

FIGS. 1A-1B illustrate a medical device including an instrument, according to aspects of this disclosure.

FIG. 2 is a cross-sectional view of the instrument of FIGS. 1A-1B and its actuation mechanism, according to aspects of this disclosure.

FIG. 3 is a cross-sectional view of another exemplary instrument and its actuation mechanism, according to other aspects of this disclosure.

FIGS. 4A-4B are, respectively, a cross-sectional view and a perspective view of another exemplary instrument and its actuation mechanism, according to other aspects of this disclosure.

FIGS. 5A-5B illustrate another exemplary instrument and its actuation mechanism, according to other aspects of this disclosure.

FIG. 5C is a cross-sectional view of an aspect of the actuation mechanism of FIG. 5B, according to aspects of this disclosure.

FIGS. 6A-6B illustrate, respectively, another exemplary instrument and its actuation mechanism, and a perspective view of the actuation mechanism, according to other aspects of this disclosure.

FIGS. 7A-7B illustrate another exemplary instrument and its actuation mechanism, according to other aspects of this disclosure.

FIGS. 7C-7D illustrate perspective views of an aspect of the actuation mechanism of FIGS. 7A-7B, according to aspects of this disclosure.

FIG. 8 illustrates another exemplary instrument and its actuation mechanism, according to other aspects of this disclosure.

FIG. 9 illustrates another exemplary instrument and its actuation mechanism, according to other aspects of this disclosure.

FIGS. 10A-10B illustrate, respectively, another exemplary instrument and its actuation mechanism, and a perspective view of an aspect of the instrument, according to other aspects of this disclosure.

## DETAILED DESCRIPTION

Reference will now be made in detail to aspects of the disclosure, examples of which are illustrated in the accompanying drawings. Wherever possible, the same or similar reference numbers will be used through the drawings to refer to the same or like parts. The term "distal" refers to a portion farthest away from a user when introducing a device into a subject (e.g., a patient). By contrast, the term "proximal" refers to a portion closest to the user when placing the device into the subject.

Both the foregoing general description and the following detailed description are exemplary and explanatory only and are not restrictive of the features, as claimed. As used herein, the terms "comprises," "comprising," "having," "including," or other variations thereof, are intended to cover a non-exclusive inclusion such that a process, method, article, or apparatus that comprises a list of elements does not include only those elements, but may include other elements not expressly listed or inherent to such a process, method, article, or apparatus. Additionally, the term "exemplary" is used herein in the sense of "example," rather than "ideal." As used herein, the terms "about," "substantially," and "approximately," indicate a range of values within +/- 5% of the stated value unless otherwise stated.

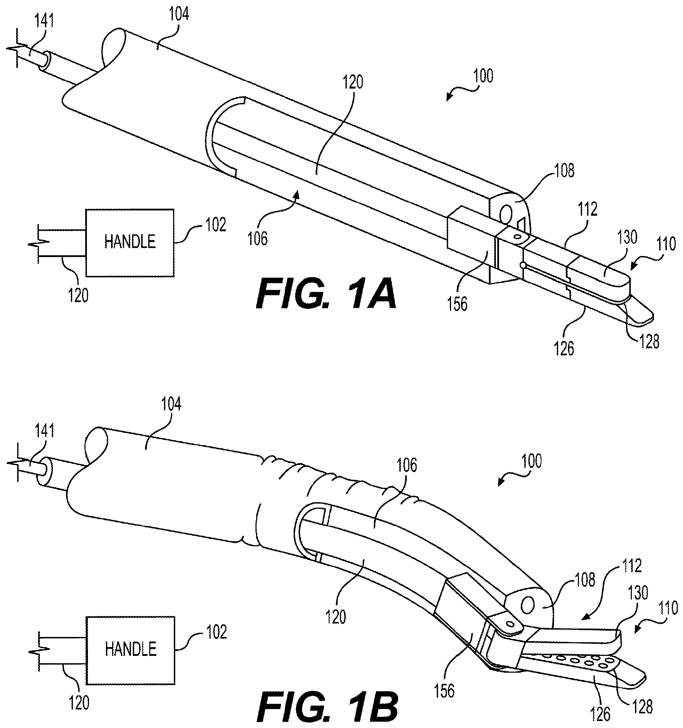

Embodiments of the disclosure may solve one or more of the limitations in the art. The scope of the disclosure, however, is defined by the attached claims and not the ability to solve a specific problem. The disclosure, in certain embodiments, is drawn to a medical device including an instrument or tool that may be actuated via a rotational force. The medical device is not particularly limited and may be any device (e.g., a tool or instrument), suitable for use in various medical procedures (e.g., bronchoscopy, duodenoscopy, endoscopy, colonoscopy, ureteroscopy). Furthermore, the medical device may be used in conjunction with robotic systems. While FIGS. 1A-10B illustrate a tissue fastener (e.g., a stapler) as the exemplary device, the device may include any suitable distal end-effector or feature that may require the pivoting of one aspect of the instrument relative to another aspect, such as, staplers, forceps, cutters, clamps, clips, or graspers. Actuation of the instrument, as used in this disclosure, may refer to the aforementioned pivoting of one aspect of the instrument relative to another.

The device may further include an actuation mechanism engaging the end-effector. The actuation mechanism includes a motor, a rotatable shaft, and a connector configured to engage the distal end-effector. The motor is not particularly limited, and may be any suitable motor configured to impart a sufficient degree of torque, i.e., a rotational force, to a coupled or connected rotatable shaft. The degree of torque is not particularly limited, and, for example, may be up to $4 \times 10^{-3}$ Nm. In accordance with the present claimed invention, the motor is positioned at a distal portion of the device, proximate to the distal end-effector. In other examples, the motor may be positioned at a proximal portion of the device (e.g., in a portion of the device that remains external to a subject during a procedure) and may engage the rotatable shaft via suitable connective means, or may be coupled to a flexible rotatable shaft extending throughout a length of the medical device. The motor may be of any appropriate size that does not interfere with the operation or the functionality of the medical device. For example, the motor may be a micromotor.

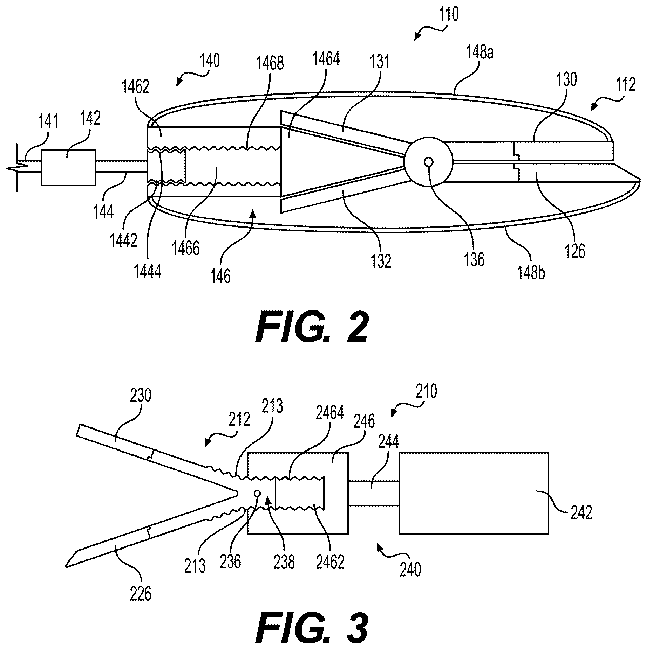

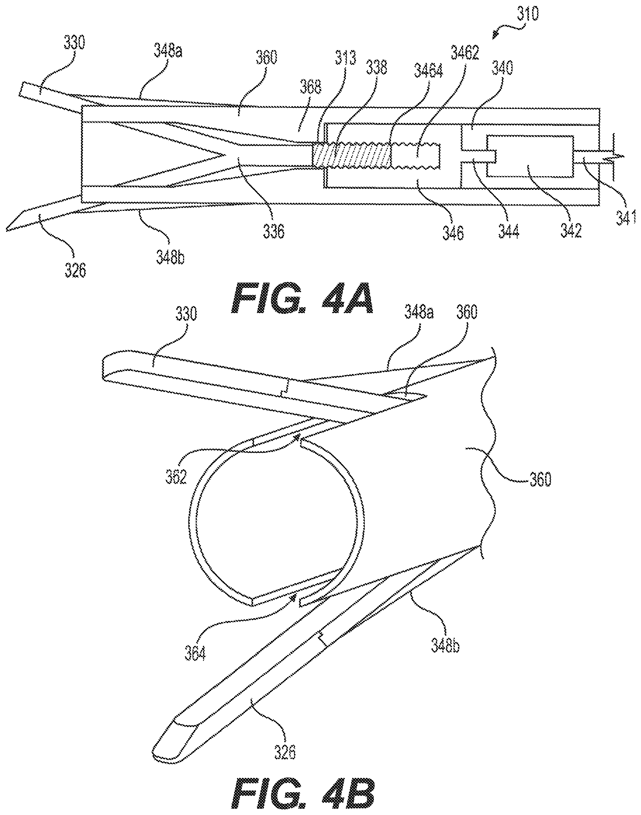

The rotatable shaft is not particularly limited, and may be any suitable shaft configured to rotate due to the rotational force imparted by the motor. For example, the rotatable shaft may include a first end (e.g., a proximal end) engaging the motor, and a second end (e.g., a distal end). The connector may be coupled or fixed to the second end of the rotatable shaft, or may be a monolithic distal aspect of the rotatable shaft. The connector may be any suitable feature that may engage a portion of the distal end-effector. Exemplary connectors may include a wedge feature positioned between two aspects of the distal end-effector (e.g., as shown in FIG. 2.), a tube or cylindrical feature including a threaded lumen engaging a threaded proximal aspect of the end-effector (e.g., as shown in FIGS. 3 and 4A), and other suitable connective features. In some other examples, the actuation mechanism may further include additional features, such as wires, cables, etc., that are coupled to the distal end-effector, and further assist in the actuation of the distal end-effector.

The medical device may further include a controller or an actuator engaging the above-discussed motor. The controller is not particularly limited, and may be any suitable controller configured to operate the motor. The controller may be located at a proximal end or portion of the device, so that it may be accessible to an operator during a procedure, while other portions of the device may be located within a patient. The means or mechanisms by which the controller engages the motor are not particularly limited and may include, e.g., connective cables, wireless connectivity, or gears. The controller may be configured to at least selectively turn on/off the motor, and may include one or more additional controls, including a control to adjust the speed at which the motor imparts rotational force, a control to select and/or change the direction of the rotation imparted by the motor, and/or other additional controls.

The rotational force imparted by the motor onto the rotatable shaft and, consequently, onto the connector may, in turn, directly or indirectly engage the pivot of the distal end-effector, thereby actuating the instrument. Aspects of the motor, shaft, connector, and/or distal end-effector may confer mechanical leverage or advantage, which may increase the force applied to the pivoting function of the distal end-effector. The medical device may further include additional features monitoring the aforementioned force applied. For example, additional features may include an: encoder, which may monitor the speed of the motor's rotary machine, which, in turn, may help assess when the pivotable aspects of the end-effector are closed or at maximum force; and force sensors, which may monitor the closing force of the pivotable aspects of the end-effector.

FIGS. 1A-1B illustrates a scope 100 and a tissue fastening device 110. Although the term scope is used herein, it will be appreciated that scope 100 is not particularly limited in application, and may be used in various procedures including endoscopy, duodenoscopy, bronchoscopy, ureteroscopy, colonoscopy, laparoscopy, or other procedures. Scope 100 encompasses devices such as duodenoscopes, endoscopes, colonoscopes, ureteroscopes, bronchoscopes, laparoscopes, sheaths, catheters, or any other suitable delivery device or medical device. Scope 100 may be used in conjunction with catheters, diagnostic or therapeutic tools, instruments or devices, or other types of medical devices. Scope 100 comprises a longitudinally extending body 104 that extends between a proximal end (not shown) and a distal end 108. Body 104 may include at least one lumen 106 extending throughout a length of body 104. A handle (not shown) may be coupled to the proximal end of body 104.

A tissue fastening device 110 may extend through lumen 106. Although a tissue fastening device 110 is referred to herein, the disclosure is not so limited and encompasses a wide variety of tools or instruments, as described above. A proximal end of tissue fastening device 112 may include a handle 102. Handle 102 may be coupled to a sheath 120 Handle 102 may be any suitable handle and may be ergonomically shaped to promote secure grip by a user. Handle 102 may be in connection or communication with actuation mechanism 140 (shown in FIG. 2), as described below. Handle 102 may include one or more controllers or actuators (not shown) for actuating various features of tissue fastening device 110 (e.g., by actuating actuation mechanism 140), as discussed above. While handle 102 is illustrated schematically and the controllers are not shown, such features may have any appropriate shape, configuration, and/or orientation without departing from the scope of the disclosure. For example, the controllers may include any one or more of buttons, dials, rockers, sliders, levers, etc., configured to actuate features of tissue fastening device 110. The controllers may also include functionality to move, deflect, bend, rotate, pivot, or otherwise adjust a distal portion of sheath 120 relative to handle. Alternatively, scope 100 and tissue fastening device 110 may share a handle that includes controls both devices. Although tissue fastening device 110 is shown and described as being used in conjunction with scope 100, tissue fastening device 110 may also be used independently of scope 100.

As shown in FIGS. 1A-1B and 2, tissue fastening device 110 may include a surgical instrument configured to engage body tissue and apply one or more surgical fasteners (e.g., staples) thereto. Tissue fastening device 110 may be used during minimally invasive surgical procedures, such as endoscopic procedures. A distal end of device 110 may include a stapler head 112, an actuation mechanism 140 coupled to or engaging a proximal portion of stapler head 112, and a shaft 141 coupled to a proximal portion of mechanism 140.

Stapler head 112 is not particularly limited, and may be any suitable stapling mechanism including at least a first element and a second element connected or joined via a pivot. For example, stapler head 112 may comprise a first arm, e.g., an anvil 130, and a second arm, e.g., a cartridge housing 126, which may be pivotably or rotatably coupled together at a pivot 136. Anvil 130 is not particularly limited, and may be any suitable stapler anvil known in the art. Likewise, cartridge housing 126 is not particularly limited, and may be any suitable stapler cartridge known in the art. Cartridge housing 126 may include one or more cartridge(s) 128 configured to carry a plurality of surgical fasteners, e.g., staples.

Stapler head 112 may further comprise a first leg 131 and a second leg 132, as shown in FIG. 2. First leg 131 may extend proximally from anvil 130, and second leg 132 may extend proximally from cartridge housing 126. First leg 131 and anvil 130 may be formed as a single, unitary structure or may be separate components fixed to one another. Second leg 132 and cartridge housing 126 may be formed as a single, unitary structure or may be separate components fixed to one another. Both first leg 131 and second leg 132 may protrude proximally from pivot 136, towards a connector 146 of actuation mechanism 140. First leg 131 may extend and protrude at a first transverse angle, e.g., 30 degrees, relative to a longitudinal axis of anvil 130. Second leg 132 may extend and protrude at a second transverse angle that is opposite (mirror image) of the first transverse angle, e.g., -30 degrees, relative to the longitudinal axis of housing 126. However, both the first transverse angle and the second transverse angle will continuously change as anvil 130 and cartridge housing 126 pivot about pivot 136. Alternatively, anvil 130 and cartridge housing 126 may form a scissors-type configuration, in which legs extend proximally from pivot 136 such that the legs are substantially parallel to anvil 130 or cartridge housing 126, respectively. In a configuration in which anvil 130 and cartridge housing 126 are adjacent or flush against one another (i.e., closed), the separation between first leg 131 and second leg 132 may define a space configured to receive a distal wedge 1464 of connector 146, discussed in further detail below. Thus, the first transverse angle and the second transverse angle may be any suitable angles so long as the radially inward-facing surfaces of first leg 131 and second leg 132 engage with wedge 1464, as described below. As discussed below, first leg 131 and second leg 132 may operate as levers. However, it is noted that other exemplary stapler heads, e.g., stapler head 312 of FIG. 3, may not include corollaries to first leg 131 and second leg 132, and may include additional features or characteristics configured to accommodate or engage with other exemplary actuation mechanisms, as discussed in further detail below.

Referring to FIG. 2, actuation mechanism 140 may include a motor 142, a rotatable shaft 144, connector 146 for engaging stapler head 112, and cables or wires 148a and 148b. As discussed above, motor 142 is not particularly limited. As shown in FIG. 2, motor 142 may be fixed at a distal portion of device 100, proximate to stapler head 112. Motor 142 is configured to impart torque (i.e., a rotational force) (in a clockwise direction and/or a counter-clockwise direction) to shaft 144, which is joined to a distal aspect of motor 142. Motor 142 causes shaft 144 to rotate about a longitudinal axis of shaft 144. The manner in which shaft 144 is joined to motor 142 is not particularly limited.

Shaft 144 may have any suitable material, shape, and dimensions. For example, shaft 144 may comprise a distal head 1442. Head 1442 may be a cylindrical feature protruding radially outwards relative to a remainder (more proximal portion) of shaft 144. Head 1442 may be a separate component joined to shaft 144 or may be a monolithic portion thereof. Head 1442 may include a threaded exterior 1444 that interfaces with connector 146, as described below.

Connector 146 may comprise a proximal tube 1462 and a distal wedge 1464. Tube 1462 and wedge 1464 may be separate components joined together or may be monolithic. Tube 1462 comprises a lumen 1466 longitudinally extending at least partially through a length of tube 1462 (along a longitudinal axis of tissue fastening device 110. Lumen 1466 may include a threaded surface 1468. Lumen 1466 and head 1442 may have dimensions (e.g., diameters) such that threaded surface 1468 interfaces with threaded exterior 1444 of head 1442. A limiting element, such as a sleeve (not shown) may inhibit tube 1462 from rotating about its longitudinal axis. Because tube 1462 is inhibited from rotating, rotation of head 1442 in a clockwise or counter-clockwise direction may result in the longitudinal translation of connector 146 in a first direction (e.g., proximally along a longitudinal axis of tissue fastening device 110) or second direction (e.g., distally along the longitudinal axis of tissue fastening device 110).

Wedge 1464 may be sized and shaped so that its outer surfaces may engage with radially inner surfaces of first leg 131 and second leg 132. For example, when anvil 130 and cartridge housing 126 are adjacent or flush against one another (i.e., closed), outer surfaces of wedge 1464 may complement an angle and size of inner surfaces of first leg 131 and second leg 132. stapler head 112

As shown in FIG. 2, first cable 148a includes a first end joined to tube 1462 (e.g., a proximal end of tube 1462) and a second end joined to a portion (e.g., a distal portion) of anvil 130. Second cable 148b includes a first end joined to tube 1462 (e.g., a proximal end of tube 1462) and a second end joined to a portion (e.g., a distal portion) of cartridge housing 126. Cables 148a and 148b may be on opposite sides of stapler head 112. Cables 148a and 148b may be of any suitable, biocompatible materials, e.g., stainless steel, nitinol, etc. It is also noted that rods may be implemented in place of cables 148a and 148b, as rods may add stiffness while providing less superfluous motion than cables.

Actuation mechanism 140 may operate via motor 142 applying torque to shaft 144, thereby rotating shaft 144 and distal head 1442 in a first direction (e.g., clockwise). Such rotation may result in a proximal translation of connector 146, relative to shaft 144 and stapler head 112, due to the threaded engagement between distal head 1442 and connector 146. Proximal translation of connector 146 may translate wedge 1464 away from stapler head 112. The proximal translation of connector 146 increases tension in cables 148a and 148b, causing them respectively pulling on anvil 130 and cartridge housing 126 in opposite directions, and thereby pivoting anvil 130 and stapler cartridge 126 away from one another about pivot 136, and transitioning stapler head 112 into an open configuration (shown in FIG. 1B). Simultaneously, first and second legs 131, 132 may be brought closer to one another. Connector 146 may be inhibited from further proximal translation via a stop (not shown) coupled to motor 142 or shaft 144.

When motor 142 rotates shaft 144 and distal head 1442 in a second direction (e.g., counter-clockwise), connector 146 may translate distally, relative to shaft 144 and stapler head 112. The distal translation of connector 146 may drive wedge 1464 into the space between first leg 131 and second leg 132 of stapler head 112. Engagement between outer surfaces of wedge 1464 and inner surfaces of first and second legs 131, 132 increasingly separates first leg 131 from second leg 132 (which act as levers on anvil 130 and stapler cartridge 126), thereby pivoting anvil 130 and/or stapler cartridge 126 towards one another about pivot 136 and transitioning stapler head 112 into a closed configuration (shown in FIG. 1A). Wedge 1464 may be inhibited from further distal translation once the distalmost point of wedge 1464 abuts again a proximal side of pivot 136. Alternatively, shape of first leg 131 and second leg 132 may inhibit wedge 1464 from moving distally passed a predetermined location.

Alternatively, stapler head 112 and/or wedge 1464 may be configured such that only one of anvil 130 and stapler cartridge 126 is movable. In such a case, wedge 1464 may have a right triangle shape, for example. In alternative examples, anvil 130 may be rotatably biased to an open configuration (FIG. 1B), e.g., biased away from cartridge housing 126, thereby defining a space between the anvil 130 and cartridge housing 126. Alternatively, anvil 130 may be rotatably biased toward the fastening (o.e., closed) configuration (FIG. 1A), e.g., biased toward the cartridge housing 126, so that anvil 130 and cartridge housing 126 may be adjacent or flush against one another. In either arrangement, anvil 130 may be moved toward or away from cartridge housing 126 and cartridge 128 via actuation mechanism 140. The application of surgical fasteners may be actuated by, or actuated subsequently after, a certain degree of pivoting of anvil 130 relative to staple cartridge housing 126 (or vice versa).

Various aspects of stapler head 112 and actuation mechanism 140 may be adjusted to, in turn, adjust the actuation force of actuator 140. Such aspects may include, the thread pitch (i.e., the distance between peaks of the threads), a gearing configuration between motor 142 and shaft 144, a tapering angle of wedge 1464, a length of legs 131 and 132, and lengths of anvil 130 and cartridge housing 126.

For example, the mechanical advantage offered by shaft 144 may be calculated by the following equation:

$$(1)\ \text{Mechanical advantage} = \frac{(\pi)(\text{diameter of threaded shaft})}{\text{pitch}}$$

Thus, for example, shaft 144 having a diameter of 0.5 mm and a pitch equal to 0.15 mm may result in a mechanical advantage of approximately 10.5. In another example, shaft 144 having a diameter of 1 mm and a pitch equal to 0.1 mm may result in actuation mechanism 140 having a mechanical advantage of approximately 31.4. This mechanical advantage may amplify the force delivered by motor 142.

Dimensions of wedge 1464 may be chosen to confer a mechanical advantage. For example, wedge 1464 may have a length (along a longitudinal axis of device 110) that is greater than a width (perpendicular to the longitudinal axis and extending between first arm 131 and second arm 132) of wedge 1464. For example, if the length of wedge 1464 is three times greater than the width of wedge 1464, wedge 1464 would confer a mechanical advantage of 3. Thus, the mechanical advantage for the wedge may be equal to a ratio of its length to its width. Such a configuration of wedge 1464 is merely exemplary, and other dimensions of wedge 1464 may be chosen to deliver a desired force and/or mechanical advantage. To maintain a compact arrangement, first leg 131 and second leg 132 may have the same length (measured between a distal end of the respective leg 131, 132 and pivot 136).

In a configuration where shaft 144 delivers a mechanical advantage of 10.5 and wedge 1464 delivers a mechanical advantage of 3, a total mechanical advantage (i.e., the mechanical advantage of shaft 144 multiplied by the mechanical advantage of wedge 1464) may be 31.5. Thus, if motor 142 delivers a force of $4 \times 10^{-5}$ Nm, i.e., an input torque, an output force of 15N may be obtained. This may be determined via the following equation:

$$(2)\ \text{Output Force} = \frac{(\pi)(2)(\text{Input Torque})}{\text{pitch}}\ (\text{mechanical advantage of wedge})$$

Configurations of the elements of stapler head 112 and actuation mechanism 140 may be chosen to deliver a desired force. Higher mechanical advantage may be advantageous as it may allow for smaller and/or faster motors to deliver a required force. A desirable force for tissue holding and fastening may range between approximately 5 N and approximately 20 N, but is not limited thereto. Elements of stapler head 112 and actuation mechanism 140 may be configured so as to provide a mechanical advantage that results in such a desired force being delivered. In examples, force may be enhanced by other means, such as anvil 130 and/or cartridge housing 126 having blunt teeth that may cause localization and amplification of the applied force.

As noted above, tissue fastening device 110 may further comprise a shaft 141 longitudinally extending through sheath 120. Shaft 141 may include a first end coupled to motor 142 and a second end coupled to handle 102 (e.g., to a controller of handle 102). Shaft 141 may, for example, include cables or wires providing a connection between controllers and actuators (not shown) of handle 102 (shown in FIGS. 1A and 1B) and motor 142.

Tissue fastening device 110 may further comprise additional features configured to sheath and or house aspects of device 110. For example, as shown in FIGS. 1A and 1B, device 110 may further include a housing 156 configured to house first leg 131 and second leg 132 of stapler head 112, and actuation mechanism 140. Housing 156 is not particularly limited and may be any suitable shape. Sheath 120 may be configured to sheath at least a portion of shaft 141, as shown in FIGS. 1A and 1B. Sheath 132 is not particularly limited and may be of any suitable material and/or dimensions (e.g., dimensions suitable to pass through lumen 106 of scope 100).

Referring to FIGS. 1A-2, an example of how scope 100 and device 110 may be used is further discussed below. A user may deliver a distal portion of body 104 of scope 100 into the body of a subject, e.g., via a natural orifice (such as a mouth or anus) and through a tortuous natural body lumen of the subject, such as an esophagus, stomach, colon, etc., towards a targeted site. Once near or adjacent to the targeted site, the user may extend tissue fastening device 110 through lumen 106 of scope 100 towards the targeted site. **If** stapler head 112 is initially in an open configuration, the user may adjust device 110 so that the targeted site is held between anchor 130 and cartridge 126 of stapler head 112. If stapler head 112 is initially in a closed configuration, the user, via at least one controller or actuator of handle 102, may operate motor 142 to transition stapler head 112 into an open configuration, via actuation mechanism 140. After capturing or holding the targeted site between anchor 130 and cartridge 126, the user, via at least one controller or actuator of handle 102, may operate motor 102 to transition stapler head 112 into the closed configuration, via actuation mechanism 140. The user may actuate the application of one or more surgical fasteners, via at least one controller or actuator of handle 102.

FIGS. 3-10B illustrate various exemplary embodiments of fastening devices, e.g., stapler heads, and actuation mechanisms. Any one of these exemplary embodiments may be used in conjunction with scope 100, including body 104 and handle 102.

FIG. 3 illustrates another exemplary tissue fastening device 210. Device 210 is similar to device 110 in some respects. Like device 110, tissue fastening device 210 may include a stapler head 212, an actuation mechanism 240 coupled to or engaging a proximal portion of stapler head 112, and a shaft (not shown) that may be coupled to a proximal portion of mechanism 210.

Like stapler head 112, stapler head 212 includes an anvil 230, a stapler cartridge housing 226 including one or more cartridges (not shown), and a pivot 236. Unlike stapler head 112, stapler head 212 further includes a proximal body 238. Proximal body 238 may be cylindrical in shape and may extend linearly away from pivot 236, along a longitudinal axis of device 210. Stapler head 212 may include a proximal threaded exterior 213. Threaded exterior 213 may surround at least a portion (e.g., the entirety) of proximal body 238, and may extend along at least a portion of a length of anvil 230 and at least a portion of a length of cartridge housing 226, as shown in FIG. 3. Proximal threaded exterior 213 interfaces with a connector 246, as discussed in further detail below.

Actuation mechanism 240, like mechanism 140, includes a motor 242, a rotatable shaft 244, and connector 246 engaging stapler head 210. Motor 242 may be similar or identical to that of motor 142. Shaft 244 is not particularly limited, and may be any suitable rod-shaped shaft coupled to motor 242 at a proximal end. The manner in which shaft 244 is joined to motor 242 is not particularly limited. The other end of shaft 244 may be coupled or connected to connector 246.

Connector 246 may be a cylindrical structure, but not limited thereto, of a greater diameter than shaft 244. Thus, connector 246 may protrude radially outwards relative to shaft 244. Connector 246 may define a lumen 2462 longitudinally extending through at least a portion of a length (along a longitudinal axis of device 210) of connector 246. Lumen 2462 includes a threaded surface 2464 that interfaces with threaded exterior 213 of stapler head 212. Stapler head 212 may be inhibited from rotating about a longitudinal axis of device 210. The means by which rotation of stapler head 212 is inhibited is not particularly limited, and may be via any suitable feature, e.g., an outer sheath 360 (having a first slot 362 and a second slot 364) as shown in FIG. 4A and described below. Therefore, rotation of head connector 246 in a clockwise or counter-clockwise direction, via the torque applied by motor 242, results in the longitudinal translation of stapler head 212 in a first (e.g., proximal) or second (e.g., distal) direction.

Thus, actuation mechanism 240 may operate via motor 242 applying torque to shaft 244, thereby rotating shaft 244 and connector 246 in a first direction (e.g., clockwise). Such rotation may result in a proximal translation of stapler head 212, relative to connector 246 and shaft 244, due to the threaded engagement between threaded exterior 213 of stapler head 212 and threaded surface 2464 of connector 246. The proximal translation of stapler 212 results in retraction of proximal body 238 and partial retraction of anvil 230 and cartridge housing 226 into lumen 2462. Radially inward forces from surfaces of lumen 2462 on anvil 230 and cartridge housing 226 thereby pivot anvil 230 and/or stapler cartridge 226 towards one another and transition stapler head 210 into a closed configuration.

Motor 242 rotating shaft 244 and connector 246 in a second direction (e.g., counter-clockwise) may result in a distal translation of stapler head 212, relative to connector 246 and shaft 244. The distal translation of stapler head 212 may result in stapler head 210 extending out of lumen 2462. In some examples, stapler head 212 may be biased towards an open configuration so that the aforementioned distal translation of stapler head 212 results in anvil 230 and stapler cartridge 226 pivoting away from one another and transitioning stapler head 212 into an open configuration. For example, stapler head 212 may include a material having shape-memory properties (e.g., nitinol) or include one or more springs or other biasing mechanisms.

The mechanical advantage of device 210 may be determined in a similar manner as discussed above, regarding device 110. For example, similar variables include a thread pitch of threaded exterior 213, and an axial translation of stapler head 212/cartridge housing 226 relative to the movement (e.g., pivoting movement) of anvil 230 and cartridge housing 226 towards each other, creating a wedge-like effect. For device 210 (and other devices disclosed herein), the mechanical advantage may be variable during operation of device 210. For example, with respect to device 210, the mechanical advantage increases as the angle between anvil 230 and cartridge housing 226 decreases, as anvil 230 and cartridge housing 226 approach each other.

Device 210 may be used in the same or similar manner as described above for device 110.

FIGS. 4A and 4B illustrate another exemplary tissue fastening device 310. Device 310 is similar to device 210 in various respects. However, unlike stapler head 212 of device 210, a threaded exterior 313 of stapler head 312 may be limited to proximal body 338, and not extend to anvil 330 or cartridge housing 326.

Furthermore, device 310 may include an outer sheath 360, sheathing actuation mechanism 340, at least a portion of shaft 341 of device 310, and at least a portion of stapler head 312. As shown in FIG. 4B, sheath 360 may include a first slot 362 and a second slot 364. Each of slots 362 and 364 may extend longitudinally from a distal end of sheath 360 to a portion of sheath 360 that is distal to actuation mechanism 340, defining a proximal edge 366. Thus, slots 362 and 364 may only extend along a distal portion of sheath 360. Slots 362 and 364 are configured to respectively accommodate the extension and retraction of anvil 330 and cartridge housing 326 therethrough. Thus, slots 362 and 364 may be diametrically opposite from one another, in accordance with anvil 330 and cartridge housing 326, and anvil 330 and cartridge housing 326 may abut against proximal edge 366, while extending and retracting (and opening and closing) within slots 362 and 364.

Sheath 360 may further include a tapered interior 368. In an example, tapered interior 368 may be proximal to edge 366, but not limited thereto. Tapered interior 368, as shown in FIG. 4A, may have a tapered inner surface so that a diameter of the passage or lumen defined by tapered interior 368 gradually decreases from a distal portion of tapered interior 368 to a proximal portion of tapered interior 368. For example, a surface of tapered interior 368 may define an approximately cone-shaped passage or lumen. Alternatively, tapered interior 368 may define a pyramid-shaped passage or lumen. Alternatively, tapered interior 368 may not extend fully around a circumference of sheath 360 and may instead include one or more wedges. A profile of tapered interior 368 enables anvil 330 and cartridge housing 326 to further (e.g., fully) close towards one another, transitioning to a closed configuration.

Like device 110, device 310 may further include a first cable (or wire) 348a and a second cable (or wire) 348b. As shown in FIGS. 4A and 4B, first cable 348a includes a first end joined to a portion of sheath 360 that is proximal to slot 362 and a second end joined to a distal portion of anvil 330. Second cable 348b includes a first end joined to a portion of sheath 360 that is proximal to slot 364 and a second end joined to a distal portion of cartridge housing 126. Cables 348a and cables 348b may be diametrically opposite from one another so that cables 348a and 348b respectively pull proximally on anvil 330 and cartridge housing 326. Cables 348a and 348b may also be of a sufficient length to allow anvil 330 and cartridge housing 326 to pivot towards and away from one another. Cables 348a and 348b may be of any suitable, biocompatible materials, e.g., stainless steel, nitinol, etc.

Like mechanism 240, actuation mechanism 340 may operate via a motor 342 applying torque to a shaft 344, thereby rotating shaft 344 and a connector 346 in a first direction (e.g., clockwise). Stapler head 312 may be inhibit from rotating about a longitudinal axis of device 310 (e.g., via slots 362 and 364 and/or cables 348a and 348b.) Thus, rotation of connector 346 may result in a proximal translation of stapler head 312, relative to connector 346 and shaft 344, due to the threaded engagement between a threaded exterior 313 of proximal body 338 and a threaded surface 3464 of connector 346. The proximal translation of stapler head 312 results in retraction of a proximal body 338 into lumen 3462 and retraction of anvil 330 and cartridge housing 326 into outer sheath 360, via slots 362 and 362, thereby pivoting anvil 330 and/or stapler cartridge 326 towards one another and transitioning stapler head 310 into a closed configuration.

Motor 342 rotating shaft 344 and connector 346 in a second direction (e.g., counter-clockwise) may result in a distal translation of stapler head 312, relative to connector 346 and shaft 344, and the extension of anvil 330 and cartridge housing 326 out of outer sheath 360. Moreover, the distal translation or extension of anvil 330 and cartridge housing 326, relative to outer sheath 360, results in cables 348a and 348b respectively pulling on anvil 330 and cartridge housing 326 in opposite directions, thereby pivoting anvil 330 and stapler cartridge 326 away from one another and transitioning stapler head 310 into an open configuration. In other examples, anvil 330 and cartridge housing 326 may be of a material having shape memory properties, e.g., nitinol, and may be biased towards an open or closed configuration. Anvil 330 and cartridge housing 326 may additionally or alternatively include springs or other feature between them to bias them to an open or closed configuration. In such an example where anvil 330 and cartridge housing 326 are biased into an open configuration, sheath 360 may limit an extent to which anvil 360 and cartridge housing 326 can open. Connector 346 may be inhibited from further distal translation via a stop (not shown) coupled to connector 346, outer sheath 360, or other aspects of actuation mechanism 340.

Mechanical advantage of device 310 may be determined in a similar manner as discussed above regarding devices 110 and 210. For example, similar variables include the thread pitch of threaded exterior 313. Other variables include an axial translation of stapler head 312/cartridge housing 326 relative to the pivoting movement of anvil 330 and cartridge housing 326 towards each other, creating a wedge-like effect. As described with respect to device 210, the mechanical advantage may vary as device 310 is operated. For example, the mechanical advantage increases as the angle between anvil 330 and cartridge housing 326 decreases as they approach each other. Tapered interior 368 may further provide a wedge-like mechanical advantage.

Device 310 may be used in the same or similar manner as described above for device 110.

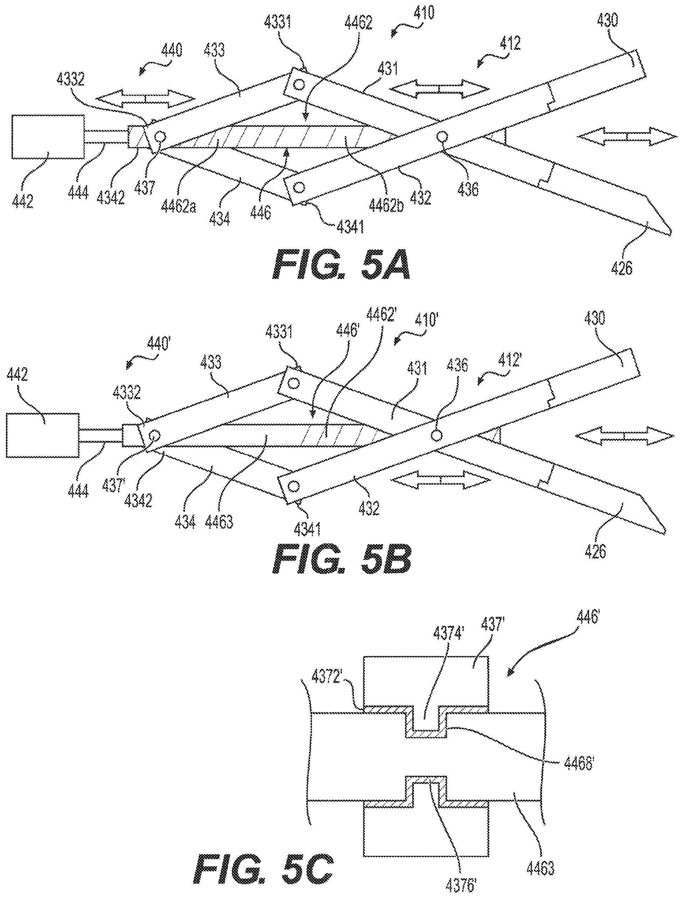

FIG. 5A illustrates another exemplary tissue fastening device 410. Device 410 is similar to devices 110 and 210 in various respects. Like stapler head 112, a stapler head 412 comprises a first leg 431 and a second leg 432, as shown in FIG. 5A. First leg 431 may extend proximally from and approximately coaxial or approximately parallel with cartridge housing 426. Second leg 432 may extend proximally from and approximately coaxial or approximately parallel with anvil 430. However, unlike stapler head 112, stapler head 412 further comprises a third leg 433 and a fourth leg 434. Third leg 433 includes a first end 4331 pivotably coupled to first leg 431 and a second end 4332 pivotably coupled to a connector 446. Similarly, fourth leg 434 includes a first end 4341 pivotably coupled to second leg 432 and a second end 4342 pivotably coupled to connector 446. Both second end 4332 and second end 4342 may be pivotably coupled to connector 446 via a coupler 437. Furthermore, pivot 436, pivotably coupling anvil 430 (and first leg 431) to cartridge housing 426 (and second leg 432) may be coupled to connector 446. Thus, as shown in FIG. 5A, legs 431, 432, 433, and 434 of stapler head 410 may form a scissor-jack like configuration.

Connector 446 may include a cylindrical rod or shaft including a threaded exterior 4462. Threaded exterior 4462 may extend along at least a portion of (e.g., throughout an entire length of) connector 446, as shown in FIG. 5A. Threaded exterior 4462 may include a first threaded portion 4462a threaded in a first direction, and a second threaded portion 4462b threaded in a second direction that is opposite of the aforementioned first direction. The lengths of first threaded portion 4462a and second threaded portion 4462 is not particularly limited, and, in some instances, may be equal lengths. Thus, in such instances, connector 446 includes two threaded portions 4462a and 4462b of equal lengths, threaded in opposite directions.

Coupler 437, pivotably coupling second ends 4332 and 4342, may be threadably engaged to first threaded portion 4462a via any suitable means, such that, when connector 446 rotates, coupler 437 may longitudinally translate across a length of first threaded portion 4462a, while remaining rotationally stationary, relative to connector 446. Coupler 437, along with second ends 4332 and 4342, may thus translate across a length of first threaded portion 4462a in a proximal and distal direction, depending on the rotation of shaft 444. Similarly, pivot 436 may be threadably engaged to second threaded portion 4462b via any suitable means, such that pivot 436 may longitudinally translate across a length of second threaded portion 4462b, while remaining rotationally stationary, relative to connector 446. Thus, pivot 436 may translate across a length of second threaded portion 4462b in a proximal and distal direction, depending on the rotation of shaft 444. The translation of coupler 437 and pivot 436 will be in opposite directions due to first threaded portion 4462a and second threaded portion 4462b being threaded in opposite directions.

An actuation mechanism 440 may operate via a motor 442 applying torque to shaft 444, thereby rotating a shaft 444 and connector 446 in a first direction (e.g., clockwise). Such rotation may result in a proximal translation of coupler 437, along with second ends 4332 and 4342, relative to connector 446, due to the threaded engagement between first threaded portion 4462a of connector 446 and coupler 437. Such rotation also results in a distal translation of pivot 436, relative to connector 446, due to the threaded engagement between second threaded portion 4462b and pivot 436. The proximal translation of coupler 437 and the distal translation of pivot 436 results in proximal ends first leg 431 and second leg 432 pivoting towards one another, and also distal ends of third leg 433 and fourth leg 434 pivoting towards one another, thereby pivoting anvil 430 and cartridge housing 426 towards one another and transitioning stapler head 412 into a closed configuration. Furthermore, anvil 430 and cartridge housing 426, while pivoting towards one another, may translate distally into a targeted tissue, which may be advantageous for certain procedures.

Motor 442 rotating shaft 444 and connector 446 in a second direction (e.g., counter-clockwise) may result in a distal translation of coupler 437, along with second ends 3332 and 3342, relative to connector 446, due to the threaded engagement between first threaded portion 4462a of connector 446 and coupler 437. Such rotation also results in a proximal translation of pivot 436, relative to connector 446, due to the threaded engagement between second threaded portion 4462b and pivot 436. The distal translation of coupler 437 and the proximal translation of pivot 436 results in first leg 431 and second leg 432 pivoting away from one another, and also third leg 433 and fourth leg 434 pivoting away from one another, thereby pivoting anvil 430 and cartridge housing 426 away from one another and transitioning stapler head 412 into an open configuration. As noted above, the translation of coupler 437 and pivot 436 in opposite directions is attributed to first threaded portion 4462a and second threaded portion 4462b being threaded in opposite directions.

FIG. 5B illustrates another exemplary tissue fastening device 410', which is similar to device 410 in various respects. However, connector 446' is without two different threaded portions, e.g., portions 4462a and 4462b as shown in FIG. 5A, as threaded exterior 4462' is limited to a distal portion of connector 446'. Alternatively, threaded exterior 4462' may extend along an entirety of connector 446'. Thus, connector 446' includes an unthreaded exterior 4463 and threaded exterior 4462', the lengths of which are not particularly limited.

Coupler 437', pivotably coupling second ends 4332 and 4342, may be fixed at a proximal position along unthreaded exterior 4463 of connector 446', while allowing the rotation of connector 446' relative to coupler 437'. For example, coupler 437' may be pivotably fixed to legs 433 and 434, while allowing connector 446' to pass through a lumen or passage of coupler 437' and movably rotate. As shown in FIG. 5C, an exemplary coupler 437' may include a lumen 4372', and an inner aspect 4374' defining an inner passage 4376'. Inner aspect 4374' may protrude radially inwards towards a longitudinal axis of coupler 437', and may extend circumferentially around the inner surface of coupler 437'. Connector 446' may include a recess or a slot 4468' extending around a circumference of unthreaded exterior 4463, which receives inner aspect 4374'. Such engagement between inner aspect 4374' and slot 4468' inhibits a longitudinal translation of coupler 437' relative to connector 446', while also allowing for the rotation of connector 446' as it extends throughout lumen 4372' and inner passage 4376'. Thus, connector 446 may be axially fixed relative to coupler 437' but may be rotatable relative to coupler 437'. Pivot 436 may be threadably engaged to threaded exterior 4462' via any suitable means, such that pivot 436 may longitudinally translate across a length of threaded exterior 4462'. While connector 446' rotates, pivot 436 may not rotate (i.e., may be rotationally fixed). Thus, pivot 436 may translate across a length of threaded exterior 4462' in a proximal and distal direction, depending on a rotation of shaft 444.

Thus, actuation mechanism 440' may operate in a similar manner as that of mechanism 440, except coupler 437' (and second ends 4332 and 4342) remains stationary (does not move longitudinally/axially and rotate). The distal translation of pivot 436, relative to connector 446', results in proximal ends first leg 431 and second leg 432 pivoting towards one another, and also distal ends of third leg 433 and fourth leg 434 pivoting towards one another, thereby pivoting anvil 430 and cartridge housing 426 towards one another and transitioning stapler head 412 into a closed configuration. The distal translation of anvil 430 and cartridge housing 426, while pivoting towards one another, may be advantageous for certain procedures. The proximal translation of pivot 436, relative to connector 446', results in first leg 431 and second leg 432 pivoting away from one another, and also third leg 433 and fourth leg 434 pivoting away from one another, thereby pivoting anvil 430 and cartridge housing 426 away from one another and transitioning stapler head 412 into an open configuration.

It is noted that in another alternate embodiment, threaded exterior 4462' may be limited to a proximal portion of connector 446', and pivot 436 may be fixed at a distal position along an unthreaded exterior of connector 446'. In such an embodiment, coupler 437 may translate across a length of threaded exterior 4462', while pivot 436 remains stationary. Thus, anvil 430 and stapler cartridge 426 may pivot towards or away from one another, due to the translation of coupler 437, while remaining axially stationary.

Devices 410 and 410' may provide mechanical advantages resulting from the translation of rotation into axial movement. Furthermore, devices 410 and 410' may provide mechanical advantages due to leverage action of stapler head 412, 412'. Although the mechanical advantages of device 410 are specifically discussed below, it will be appreciated that these advantages also apply to device 410'. The mechanical advantage of device 410 may depend on variables including the thread pitch of threaded exterior 4462, the axial translation of stapler head 412, the scissoring and leverage action of anvil 430 and cartridge housing 426 towards each other, and the angle between anvil 430 and cartridge housing 426. For example, the half angle between anvil 430 and cartridge housing 426 (also the angle between third leg 433 and connector 446) must be greater than 45° for device 410 to offer a mechanical advantage. Thus, the mechanical advantage of device 410 (and 410') may increase as the angle between anvil 430 and cartridge housing 426 increases as they separate from each other.

In an alternative configuration of devices 410 and 410', first leg 431 may extend transversely and proximally from anvil 430 and second leg 432 may extend transversely and proximally from cartridge hosing 426. Thus, each of (a) first leg 431 and anvil 430 and (b) second leg 432 and cartridge housing 426 may define V-shaped features, in such an exemplary embodiment. In such an embodiment, mechanical advantage may increase as the angle between anvil 430 and cartridge housing 426 decreases as they translate towards one another.

Devices 410, 410' may be used in the same or similar manner as described above for device 110.

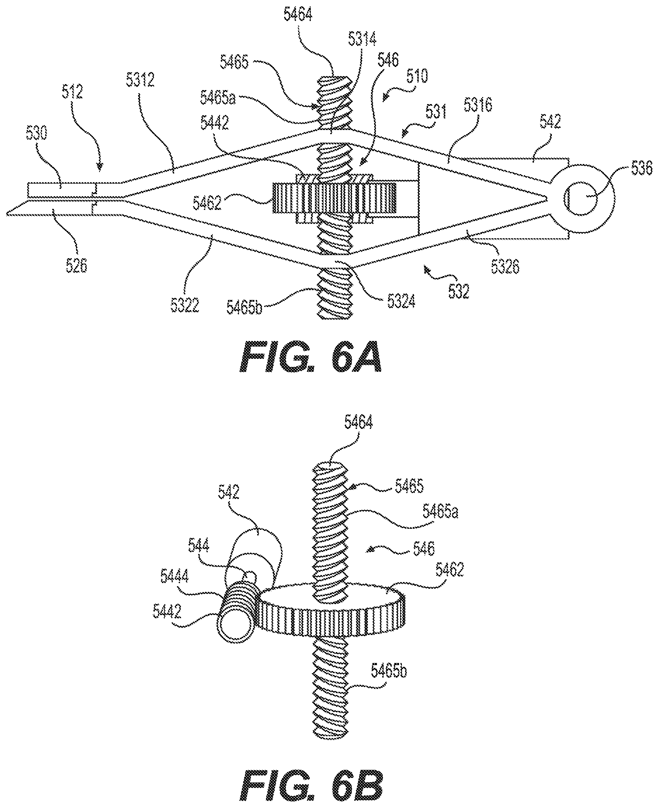

FIG. 6A illustrates another exemplary tissue fastening device 510. Device 510 is similar to devices 110. Like, shaft 144, shaft 544 comprises a distal head (e.g., a worm gear) 5442 including a threaded exterior 5444 that interfaces with connector 546. Distal head 5442 may be any suitable dimension, such as a dimension small enough to fit through lumen 136 of scope 100 (e.g., up to 0.5 mm in length). Unlike connectors 146 and 446, connector 546 comprises a gear 5462 and bar 5464.

Gear 5462 is not particularly limited, and may be any suitable gear (e.g., a round, circular gear, such as a worm wheel) of a suitable size. For example, gear 5462 may be sized so as to be able to fit within lumen 106 of scope 100 (e.g., up to 1.25 mm in diameter). Gear 5462 may include a plurality of teeth. As shown in FIGS. 6A and 6B, gear 5462 may be situated so that a central, rotational axis of gear 5462 is perpendicular to a longitudinal axis of shaft 544 and distal head 5442. The teeth of gear 5462 may engage threaded exterior 5444 of head 5442 such that the rotation of head 5442 may, in turn, rotate gear 5462 about its central, rotational axis. Bar 5464 may be fixed to and extend through the center of gear 5462 so that bar 5464 longitudinally extends along the central, rotational axis of gear 5462. The length of bar 5464 is not particularly limited, e.g., less than or equal to approximately 5 mm or less than or equal to approximately 3 mm, and the position of gear 5462 along the length of bar 5464 is also not particularly limited, e.g., midway bar 5464. Bar 5464 may comprise a threaded exterior 5465. Threaded exterior 5465 may comprise a first threaded portion 5465a and a second threaded portion 5465b (see FIG. 6B), which are threaded in opposite directions from one another.

A stapler head 512 includes a stapler anvil 530 and a cartridge housing 526 (see FIG. 6A), which are pivotably coupled via pivot 536. Stapler head 512 further includes first leg 531 extending between anvil 530 and pivot 536, and second leg 532 extending between cartridge housing 526 and pivot 536. First leg 531 includes a first portion 5312, an intermediary portion 5314, and a second portion 5316, and second leg 532, likewise, includes a first portion 5322, an intermediary portion 5324, and a second portion 5326. As shown in FIG. 6A, first portions 5312 and 5322 may extend proximally, respectively, from anvil 530 and cartridge housing 526 at opposite transverse angles, relative to a longitudinal axis of stapler head 512. Likewise, second portions 5316 and 5326 may extend distally from pivot 536 at opposite transverse angles toward pivot 536, relative to the longitudinal axis of stapler head 512. Intermediary portions 5314 and 5324 may extend between the respective first and second portions, and may be approximately parallel relative to the longitudinal axis of stapler head 512. Each of intermediary portions 5314 and 5324 may include a central threaded passage, such as a hole) (not shown) configured to threadably engage bar 5464, and through which bar 5464 may extend. The central passages of intermediary portions 5314 and 5324 threadably engage bar 5464 so that intermediary portions 5314 and 5324 translate across the length of bar 5464 while also remaining rotationally stationary.

Actuation mechanism 540 may operate via a motor 542 applying torque to shaft 544, thereby rotating shaft 544 and distal head 5442 in a direction (clockwise or counter-clockwise). Such rotation results in the rotation of gear 5462, given the engagement between the teeth of gear 5462 and distal head 5442, and the rotation of threaded bar 5464, which is fixed to gear 5462. The rotation of bar 5464 may result in the translation of intermediary portion 5314 in a first (e.g., radially outward or radially inward) direction, due to the threaded engagement between first threaded portion 5465a and the central passage of intermediary portion 5314. Such rotation of bar 5464 also results in the translation of intermediary portion 5324 in a second (e.g., radially outward or radially inward) direction that is diametrically opposite of the first direction, due to the threaded engagement between second threaded portion 5465b and the central passage of intermediary portion 5324. The translation of intermediary portions 5314 and 5324 in opposite directions is attributed to first threaded portion 5465a and second threaded portion 5465b being threaded in opposite directions.

As intermediary portions 5314 and 5324 translate away from one another, stapler anvil 530 and cartridge housing 526, likewise, separate away from one another, thereby transitioning stapler head 512 to an open configuration. In contrast, as intermediary portions 5314 and 5324 translate towards one another, stapler anvil 530 and cartridge housing 526, likewise, translate toward one another, thereby transitioning stapler head 512 to a closed configuration. Thus, transitioning stapler head 512 from a closed configuration to an open configuration, and vice versa, may depend on the direction in shaft 544 and head 5442 is rotated.

As discussed above, various aspects of stapler head 512 and actuation mechanism 540 may be adjusted to, in turn, adjust the pivoting force (or closure force) of anvil 530 and cartridge housing 526 relative to the other. Such aspects may include, the thread pitch of various aspects, e.g., bar 5464, the gearing between motor 542 and shaft 544, the positioning of pivot 536, and the lengths of legs 531 and 532. Moreover, the aforementioned aspects may also help determine the mechanical advantage offered by device 510. For example, when distal head 5442 has a length of 0.5 mm and gear 5462 has a diameter of 1.25 mm (and assuming gear 5462 has approximately 24 teeth and 50% efficiency), gear 5442 may have a mechanical advantage of approximately 12. The mechanical advantage of threaded bar 5464 having a diameter of 0.5 mm and a thread pitch of 0.15 mm may be approximately 10.5, per equation 1), discussed above. It is further noted that the mechanical advantage attributed by aspects of stapler head 512, i.e., stapler anvil 530, cartridge housing 526, first leg 531, and second leg 532, may be less than 1. This may be attributed to the load position and effort position relative to pivot 536. To mitigate this, it may be preferable, in some instances, to have intermediary portions 5314 and 5324 (and bar 5464) be closer to anvil 530 and cartridge housing 526 than to pivot 536. Such modification may result in stapler head 512 offering a mechanical advantage of approximately 0.8 (a leverage ratio of (a) a distance between pivot 536 and threaded bar 5464 to (b) a distance from pivot 536 to a distal end of device 510). This would results in an overall mechanical advantage of (mechanical advantage of worm gear 5442) x (mechanical advantage of threaded bar 5464) x (mechanical advantage of stapler head 512)=(12) x (10.5) x (0.8) = 101.

Device 510 may be used in the same or similar manner as described above for device 110.

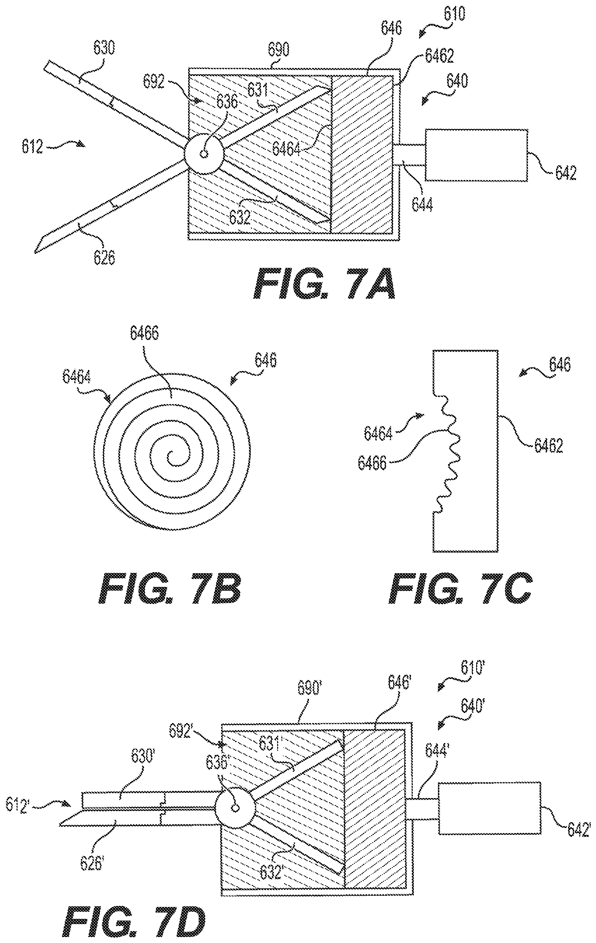

FIGS. 7A-7C illustrate aspects of another exemplary tissue fastening device 610. Device 610 is similar to device 110 in various respects. However, unlike connector 146 of device 110, a connector 646 includes a first side 6462 coupled or fixed to a distal end of a shaft 644 and a second side 6464 including a spiraling threaded exterior 6466 (shown in FIGS. 7B and 7C). The manner by which spiral threaded exterior 6466 is formed is not particularly limited. In some instances, exterior 6466 may be formed via a mold, via the removal of an extruded cut, etc. The extruded cut may be partially spherical, resulting in a depressed or concave exterior 6466, as shown in FIG. 7D. Concave exterior 6466 may accommodate for a first leg 631 and a second leg 632, as the legs pivot towards one another. A size of connector 646 may be such that device 610 may be passed through lumen 106 of scope 100. For example, a diameter of connector 646 may be less than or equal to approximately 5 mm or less than or equal to approximately 3 mm. As shown in FIG. 7A, in some instances, connector 646 may be cylindrical and of a larger diameter than shaft 644.

Spiral threaded exterior 6466 is configured to engage the proximal ends of first leg 631 and second leg 632 of stapler head 612. Such engagement may be via any suitable means. For example, the proximal ends of legs 631 and 632 may be shaped or molded to fit in the threads of exterior 6466, or the proximal ends of legs 631 and 632 may include additional features configured to engage exterior 6466.

Thus, actuation mechanism 640 may operate via motor 642 applying torque to shaft 644, thereby rotating shaft 644 and connector 646 in a direction (clockwise or counter-clockwise). Depending on the direction of rotation, such rotation results in first leg 631 and second leg 632 either moving towards or away from one another, given the engagement between the proximal ends of legs 631 and 632 and spiraling threaded exterior 6466. The proximal ends of first leg 631 and second leg 632 may ride in the grooves of threaded exterior 642, causing stapler head 612 to pivot about axis 636. First leg 631 and second leg 632 moving towards one another results in anvil 630 and cartridge housing 626 also pivoting towards one another, thereby transitioning stapler head 612 into a closed configuration. In contrast, first leg 631 and second leg 632 moving away from one another results in anvil 630 and cartridge housing 626 also pivoting away from one another, thereby transitioning stapler head 612 into an open configuration. As shown in FIG. 7A, device 610 may further include a housing 690 encasing at least a portion of actuation mechanism 640 and at least a portion of stapler head 612. Housing 690 may include a slot or passage 692, through which remaining aspects of stapler head 612 and/or actuation mechanism 640 may extend therethrough. Housing 690 is not particularly limited, and may be configured to prevent stapler head 612 from rotating, as connector 646 rotates, and hold stapler head 612 in place at pivot 636.

FIG. 7D illustrates aspects of another exemplary tissue fastening device 610', which may be similar to device 610 in various respects. Unlike device 610, device 610' may be designed such that when anvil 630' and cartridge housing 626' are adjacent and in a closed configuration, the proximal ends of first leg 631' and second leg 632' may be positioned along an outer diameter of threaded exterior 6466. This arrangement may be beneficial in some instances, as the mechanical advantage may increase as stapler head 612' transitions into a closed configuration due to the angle between first leg 631' and second leg 632' increasing.

As discussed above, various aspects of stapler head 612 and actuation mechanism 640 may be adjusted to, in turn, adjust the pivoting force (or closure force) of anvil 630 and cartridge housing 626 relative to the other. Such aspects may include, the thread pitch of spiraling threaded exterior 6466, the incorporation of additional gears, e.g., a worm wheel, worm gear, etc., the shape of the proximal ends of legs 631 and 632, positioning of the axis of rotations of legs 631 and 632, and the lengths of legs 631 and 632. Moreover, the aforementioned aspects may also be modified to improve the mechanical advantage offered by device 610. For example, the length of anvil 630 or cartridge housing 626 may be shorter than the length of first leg 631 or second leg 632 to improve mechanical advantage. Moreover, a mechanical advantage will be at a maximum when legs 631 and 632 are along the outer most diameter of spiraling threaded exterior 6466. For example, a threaded exterior 6466 having an outer diameter of 2 mm and a pitch of 0.15 mm may offer a mechanical advantage of 42, given that $(\pi)(2mm)/(0.15mm) = 42$. Meanwhile, mechanical advantage will be at a minimum when legs 631 and 632 are along the inner most diameter of spiraling threaded exterior 6466. For example, a threaded exterior 6466 having an inner diameter of 0.4 mm and a pitch of 0.15 mm may offer a mechanical advantage of 8.4, given that $(\pi)(0.4 mm)/(0.15mm) = 8.4$.

Device 610 may be used in the same or similar manner as described above for device 110.

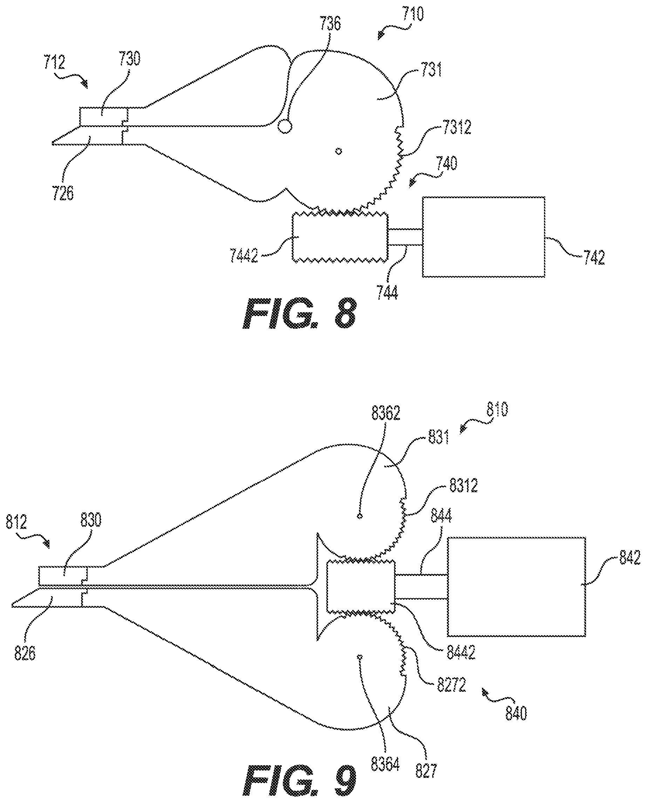

FIG. 8 illustrates another exemplary tissue fastening device 710. Device 710 is similar to device 110 in various respects. Actuation mechanism 740, like actuation mechanism 140, includes a motor 742 and a rotatable shaft 744, including a distal head 7442. Distal head 7442 is not particularly limited, and may be any suitable aspect including a plurality of threads or teeth, e.g., a worm gear, shaft, etc. However, unlike actuation mechanism 140, mechanism 740 may be without a connector, as distal head 7442 directly engages a stapler head 712.

Stapler head 712 includes a stapler anvil 730, a cartridge housing 726, and a pivot 736 pivotably coupling proximal portions of anvil 730 and cartridge housing 726. As shown in FIG. 8, cartridge housing may include a rounded proximal aspect 731 including a geared exterior 7312 extending along at least a portion of the surface of proximal aspect 731. Geared exterior 7312 may include a plurality of teeth configured to engage the plurality of teeth or threads distal head 7442, thereby enabling the pivoting of anvil 730, relative to cartridge housing 726, via the rotation of distal head 7442. It is noted that anvil 730 may be configured to remain stationary, relative to cartridge housing 726. Alternatively, anvil 730 may include rounded proximal aspect 731 (not shown), and cartridge housing 726 may remain stationary.

Thus, actuation mechanism 740 may operate via motor 742 applying torque to shaft 744, thereby rotating shaft 744 and distal head 7442 in a direction (clockwise or counter-clockwise). Such rotation results in the pivoting of stapler anvil 730, relative to cartridge housing 726, given the engagement between geared exterior 7312 and distal head 7442. Thus, for example, the rotation of distal head 7442 in a clockwise direction may cartridge housing 726 away from anvil 730, thereby transitioning a stapler head 712 into an open configuration. In contrast, the rotation of distal head 7442 in a counter-clockwise direction may pivot cartridge housing 726 towards stationary anvil 730, thereby transitioning stapler head 712 into a closed configuration. As discussed above with respect to gear 5442 of device 510, mechanical advantage of actuation mechanism 740, including e.g., shaft 744, may depend on the number of teeth of geared exterior 7312, the number of teeth of shaft 744, and the degree of efficiency.

FIG. 9 illustrates another exemplary tissue fastening device 810, which is similar to device 710 in various respects. However, a stapler head 812 differs from stapler head 712 of device 710, in that both an anvil 830 and a cartridge housing 826 engage a distal head 8442. Anvil 830 includes a rounded proximal aspect 831 including a geared exterior 8312 extending along at least a portion of the surface of proximal aspect 831. Likewise, cartridge housing 826 includes a rounded proximal aspect 827 including a geared exterior 8272 extending along at least a portion of the surface of proximal aspect 827. Thus, as shown in FIG. 9, geared exteriors 8312 and 8272 may engage opposite sides of a distal head 8442 of shaft 844 (positioned between proximal aspects 827 and 831), thereby enabling the pivoting of both anvil 830 and cartridge housing 826, relative to one another, via the rotation of distal head 8442. Anvil 830 and cartridge housing 826 may have their own respective pivots 8362 and 8364, enabling the separate (but simultaneous) pivoting of both features.

Thus, actuation mechanism 840 may operate via a motor 842 applying torque to a shaft 844, thereby rotating shaft 844 and distal head 8442 in a direction (clockwise or counter-clockwise). Such rotation results in the pivoting of both stapler anvil 830 and cartridge housing 826 towards or away from one another, given the respective engagements of geared exteriors 8312 and 8272, with distal head 8442. Thus, for example, the rotation of distal head 8442 in a clockwise direction may pivot anvil 830 and cartridge housing 826 away from one another, thereby transitioning stapler head 812 into an open configuration. In contrast, the rotation of distal head 8442 in a counter-clockwise direction may pivot anvil 830 and cartridge housing 826 towards one another, thereby transitioning stapler head 812 into a closed configuration.

Devices 710 and 810 may be used in the same or similar manner as described above for device 110.

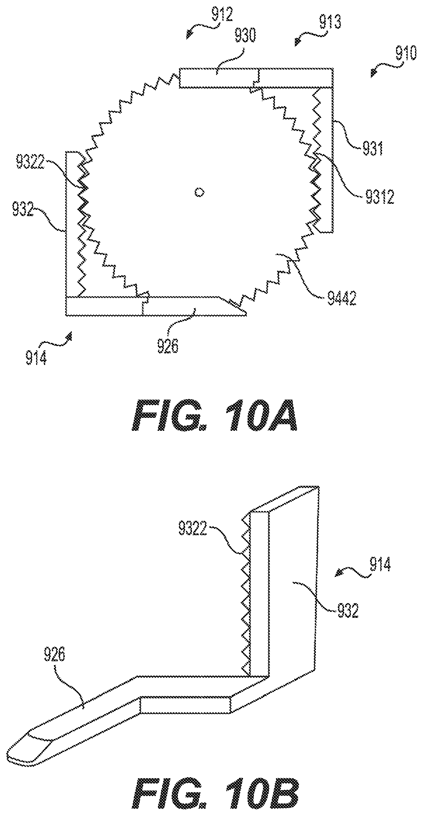

FIGS. 10A-10B illustrate another exemplary tissue fastening device 910. Device 910 is similar to device 110 in various respects. Actuation mechanism 940, like actuation mechanism 140, includes a motor (not shown) and a rotatable shaft (not shown), including a distal head 9442. Distal head 9442 is not particularly limited, and may be any suitable round or circular gear including a plurality of teeth. Distal head 9442 may directly engage a stapler anvil 930 and a cartridge housing 926.

Unlike device 110, stapler head 912 includes a first stapling aspect 913 and a second stapling aspect 914, which are separated from one another in an open configuration, as shown in FIG. 10A. First stapling aspect 913 includes an arm, i.e., anvil 930, and a leg 931, and second stapling aspect 914 includes an arm, i.e., cartridge housing 926, and a leg 932. Anvil 930 and housing 926 each include an end that is coupled to legs 931, 932. Legs 931 and 932 extend in a perpendicular direction, relative to the longitudinal axes of anvil 930 and housing 926, towards one another, thereby defining L-shaped features. Leg 931 includes a geared exterior 9312 configured to engage distal head 9442, extending along the side or surface of leg 931 that is facing distal head 9442. Likewise, leg 932 includes a geared exterior 9322 configured to engage distal head 9442, extending along the side or surface of leg 932 that is facing distal head 9442.

Leg 931 and leg 932 are positioned along opposite sides of distal head 9442, so that anvil 930 and cartridge housing 926 face one another and are approximately parallel relative to other. It is further noted that leg 932 may not be along the same plane as cartridge housing 926, and is, instead, off-plane. Likewise, leg 931 may also not be along the same plane as anvil 930. Alternatively, legs 931 and 932 may be of a wider dimension (into the page, as shown in FIG. 9B), than anvil 930/housing 926, so that anvil 930 and housing 926. Such configurations may allow anvil 930 and cartridge housing 926 to translate towards or away from one another, without any obstruction by distal head 9442, as distal head 9442 rotates and engages geared exteriors 9312 and 9322.

Thus, actuation mechanism 940 may operate via the motor (not shown) applying torque to the shaft (not shown), thereby rotating the shaft and distal head 9442 in a direction (clockwise or counter-clockwise). Such rotation results in the translation of legs 931 and 932 in an upward or downward direction of FIG. 8A, given the respective engagements of geared exteriors 9312 and 9322, with distal head 9442. Thus, anvil 930 and cartridge housing 926 move towards or away from one another,. Thus, for example, the rotation of distal head 9442 in a clockwise direction may anvil 930and cartridge housing 926 towards one another, thereby transitioning stapler head 912 into a closed configuration. In contrast, the rotation of distal head 9442 in a counter-clockwise direction may translate anvil 930and cartridge housing 926 away from one another, thereby transitioning stapler head 912 into an open configuration.

Device 910 may be used in the same or similar manner as described above for device 110.

It will be apparent to those skilled in the art that various modifications and variations may be made in the disclosed devices and methods without departing from the scope of the disclosure. Other aspects of the disclosure will be apparent to those skilled in the art from consideration of the specification and practice of the features disclosed herein. It is intended that the specification and examples be considered as exemplary only.

The invention is defined by the following claims.

**Claims**

1.   A medical device, comprising:

an end-effector, including a first feature and a second feature, wherein the first feature and the second feature are pivotably joined to one another; and

a motor (142, 242, 342, 442, 542, 642, 742, 842) coupled to a rotatable shaft (144, 244, 744), wherein the motor (142, 242, 342, 442, 542, 642, 742, 842) is arranged at distal portion of the device proximate the end effector and wherein the distal portion proximate the end effector is configured to extend within a body of a subject during a medical procedure,

wherein the rotatable shaft (144, 244, 744) engages the end-effector, and wherein the motor (142, 242, 342, 442, 542, 642, 742, 842) is configured to rotate the rotatable shaft (144, 244, 744) in a first direction and a second direction, wherein the second direction is opposite of the first direction;

wherein a first rotation of the rotatable shaft (144, 244, 744) in the first direction is configured pivot the first feature and the second feature towards one another, and

wherein a second rotation of the rotatable shaft (144, 244, 744) in the second direction is configured to pivot the first feature and the second feature away from one another.

2. The medical device of claim 1, further comprising a connector (146, 246, 346, 446, 546, 646) coupled to the rotatable shaft (144, 244, 744), wherein the connector (146, 246, 346, 446, 546, 646) is configured to directly engage the end effector.

3. The medical device of claim 2, further comprising:

a first cable (148a), including a first end coupled to the connector (146) and a second end coupled to the first feature; and

a second cable (148b), including a first end coupled to the connector (146) and a second end coupled to the second feature;

wherein the connector (146) includes a threaded lumen,

wherein the rotatable shaft (144) includes a threaded exterior, engaging the threaded lumen of the connector (146), and

wherein the first rotation of the rotatable shaft (144) in the first direction translates the connector (146) in a first longitudinal direction, and wherein the second rotation of the rotatable shaft (144) in the second direction translates the connector (146) in a second longitudinal direction.

4. The medical device of claim 3, wherein a first translation of the connector (146) in the first longitudinal direction pivots the first feature and the second feature towards one another, and wherein the second translation of the connector (146) in the second longitudinal direction causes the first cable (148a) to pull on the first feature and the second cable (148b) to pull on the second feature, thereby pivoting the first feature and the second feature away from another.

5. The medical device of claim 2, wherein the connector (246, 346, 446) includes a threaded lumen,

wherein the end-effector further includes a body proximal to the first feature and the second feature, wherein the body includes a threaded exterior,

wherein the threaded lumen of the connector (246, 346, 446) engages the threaded exterior of the body of the end-effector, and

wherein the first rotation of the rotatable shaft (244, 344, 344) in the first direction translates the end-effector in a first longitudinal direction, relative to the connector (246, 346, 446), and wherein second rotation of the rotatable shaft (244, 344, 344) in the second direction translates the end-effector in a second longitudinal direction, relative to the connector (244, 344, 344).

6. The medical device of claim 5, wherein the first feature includes a first threading and the second feature includes a second threading,

wherein the threaded lumen of the connector (346) engages the first threading of the first feature and the second threading of the second feature, and

wherein a first translation of the end-effector in the first longitudinal direction pivots the first feature and the second feature towards one another, and wherein a second translation of the end-effector in the second longitudinal direction pivots the first feature and the second feature away from another.

7. The medical device of claims 5 or 6, further comprising:

a sheath (360), sheathing at least a portion of the connector (346) and at least a portion of the end-effector, wherein the sheath (360) includes a first longitudinal slot through which the first feature of the end-effector extends and retracts, and a second longitudinal slot through which the second feature of the end-effector extends and retracts;

a first cable (348a), including a first end coupled to the sheath (360) and a second end coupled to the first feature; and

a second cable (348b), including a first end coupled to the sheath (360) and a second end coupled to the second feature;

wherein a first translation of the end-effector in the first longitudinal direction pivots the first feature and the second feature towards one another, and wherein a second translation of the end-effector in the second longitudinal direction pivots the first feature and the second feature away from another.

8. The medical device of claim 2, wherein the connector (446) comprises a cylindrical rod, and wherein at least a portion of the cylindrical bar includes a threaded exterior (4462), and

wherein the end-effector engages the threaded exterior, such that (a) the first rotation of the shaft in the first direction causes the end-effector to longitudinally translate along the threaded exterior in a first longitudinal direction, and (b) the second rotation of the shaft in the second direction causes the end-effector to longitudinally translate along the threaded exterior in a second longitudinal direction.

9. The medical device of claim 2, wherein the connector (546) includes an intermediary gear (5462) and a threaded bar (5464), wherein the threaded bar (5464) includes (a) a first threaded portion (5465a) fixed to and extends from a center of a first side of the intermediary gear, and (b) a second threaded portion (5465b) fixed to and extending from the center of a second side of the intermediary gear,

wherein the rotatable shaft (544) includes a threaded exterior engaging the intermediary gear,
wherein the first feature of the end effector engages the first threaded portion (5465a) of the threaded bar (5464), and wherein the second feature of the end effector engages the second threaded portion (5465b) of the threaded bar (5464).

10. The medical device of claim 9, wherein the first threaded portion (5465a) is threaded in a first direction and the second threaded portion (5465b) is threaded in a second direction, and wherein the second direction is opposite of the first direction.

11. The medical device of claim 3, wherein the connector includes a first side coupled to the rotatable shaft (644), and a second side including a spiral thread (6466) formed thereon,
Wherein the first feature of the end-effector includes a first end engaging a groove of the spiral thread, and wherein the second feature of the end-effector includes a second end engaging the groove of the spiral thread (6466).

12. The medical device of claim 1, wherein the rotatable shaft (744, 844) includes a threaded exterior, and
wherein the second feature of the end-effector includes a proximal surface including a first plurality of teeth (7312) engaging the threaded exterior of the rotatable shaft (744, 844).

13. The medical device of claim 12, wherein the first feature of the end-effector includes a proximal surface including a second plurality of teeth (8272) engaging the threaded exterior of the rotatable shaft (44), and wherein the rotatable shaft is positioned between the proximal surface of the first feature and the proximal surface of the second feature.

14. The medical device of any of the preceding claims, further comprising a controller in communication with the motor (142, 242, 342, 442, 542, 642, 742, 842), wherein the controller is configured to select and/or change a direction of the rotation of the shaft imparted by the motor (142, 242, 342, 442, 542, 642, 742, 842).

15. The medical device of any of the preceding claims, wherein the end-effector is a stapler (212), wherein the first feature includes an anvil (130, 230, 330, 430, 530, 630, 730, 830, 930, 930a) of the stapler (212), and wherein the second feature includes a cartridge housing (126, 226, 326, 426, 526, 626, 726, 826, 926) of the stapler (212).

**Patentansprüche**

1. Medizinische Vorrichtung, die aufweist:

einen Endeffektor, der ein erstes Merkmal und ein zweites Merkmal aufweist, wobei das erste Merkmal und das zweite Merkmal schwenkbar miteinander verbunden sind; und

einen Motor (142, 242, 342, 442, 542, 642, 742, 842), der mit einem drehbaren Schaft (144, 244, 744) gekoppelt ist, wobei der Motor (142, 242, 342, 442, 542, 642, 742, 842) an einem distalen Abschnitt der Vorrichtung in der Nähe des Endeffektors angeordnet ist und wobei der distale Abschnitt in der Nähe des Endeffektors konfiguriert ist, sich während eines medizinischen Eingriffs innerhalb eines Körpers eines Patienten zu erstrecken, wobei der drehbare Schaft (144, 244, 744) mit dem Endeffektor in Eingriff steht, und wobei der Motor (142, 242, 342, 442, 542, 642, 742, 842) konfiguriert ist, den drehbaren Schaft (144, 244, 744) in einer ersten Richtung und einer zweiten Richtung zu drehen, wobei die zweite Richtung der ersten Richtung entgegengesetzt ist;

wobei eine erste Drehung des drehbaren Schafts (144, 244, 744) in der ersten Richtung konfiguriert ist, das erste Merkmal und das zweite Merkmal zueinander zu schwenken, und

wobei eine zweite Drehung des drehbaren Schafts (144, 244, 744) in der zweiten Richtung konfiguriert ist, erste Merkmal und das zweite Merkmal voneinander weg zu schwenken.

2. Medizinische Vorrichtung nach Anspruch 1, die ferner einen Verbinder (146, 246, 346, 446, 546, 646) aufweist, der mit dem drehbaren Schaft (144, 244, 744) gekoppelt ist, wobei der Verbinder (146, 246, 346, 446, 546, 646) konfiguriert ist, direkt mit dem Endeffektor in Eingriff zu treten.

3. Medizinische Vorrichtung nach Anspruch 2, die ferner aufweist:

ein erstes Kabel (148a), das ein erstes Ende, das mit dem Verbinder (146) gekoppelt ist, und ein zweites Ende aufweist, das mit dem ersten Merkmal gekoppelt ist; und

ein zweites Kabel (148b), das ein erstes Ende, das mit dem Verbinder (146) gekoppelt ist, und ein zweites Ende aufweist, das mit dem zweiten Merkmal gekoppelt ist;

wobei der Verbinder (146) ein mit einem Gewinde versehenes Lumen aufweist,

wobei der drehbare Schaft (144) ein mit einem Gewinde versehenes Äußeres aufweist, das mit dem mit einem Gewinde versehenen Lumen des Verbinders (146) in Eingriff steht, und

wobei die erste Drehung des drehbaren Schafts (144) in der ersten Richtung den Verbinder (146) in einer ersten Längsrichtung verschiebt, und wobei die zweite Drehung des drehbaren Schafts (144) in der zweiten Richtung den Verbinder (146) in einer zweiten Längsrichtung verschiebt.

4. Medizinische Vorrichtung nach Anspruch 3, wobei eine erste Verschiebung des Verbinders (146) in der ersten Längsrichtung das erste Merkmal und das zweite Merkmal zueinander hin schwenkt, und wobei die zweite Verschiebung des Verbinders (146) in der zweiten Längsrichtung bewirkt, dass das erste Kabel (148a) an dem ersten Merkmal zieht und das zweite Kabel (148b) an dem zweiten Merkmal zieht, wodurch das erste Merkmal und das zweite Merkmal voneinander weg geschwenkt werden.

5. Medizinische Vorrichtung nach Anspruch 2, wobei der Verbinder (246, 346, 446) ein mit einem Gewinde versehenes Lumen aufweist,

wobei der Endeffektor ferner einen Körper aufweist, der sich proximal zu dem ersten Merkmal und dem zweiten Merkmal befindet, wobei der Körper ein mit einem Gewinde versehenes Äußeres aufweist,

wobei das mit einem Gewinde versehene Lumen des Verbinders (246, 346, 446) mit dem mit Gewinde versehenen Äußeren des Körpers des Endeffektors in Eingriff steht, und

wobei die erste Drehung des drehbaren Schafts (244, 344, 344) in der ersten Richtung den Endeffektor in einer ersten Längsrichtung relativ zu dem Verbinder (246, 346, 446) verschiebt, und wobei die zweite Drehung des drehbaren Schafts (244, 344, 344) in der zweiten Richtung den Endeffektor in einer zweiten Längsrichtung relativ zu dem Verbinder (244, 344, 344) verschiebt.

6. Medizinische Vorrichtung nach Anspruch 5, wobei das erste Merkmal ein erstes Gewinde aufweist und das zweite Merkmal ein zweites Gewinde aufweist,

wobei das mit einem Gewinde versehene Lumen des Verbinders (346) mit dem ersten Gewinde des ersten Merkmals und dem zweiten Gewinde des zweiten Merkmals in Eingriff steht, und

wobei eine erste Verschiebung des Endeffektors in der ersten Längsrichtung das erste Merkmal und das zweite Merkmal zueinander hin schwenkt, und wobei eine zweite Verschiebung des Endeffektors in der zweiten Längsrichtung das erste Merkmal und das zweite Merkmal voneinander weg schwenkt.

7. Medizinische Vorrichtung nach Anspruch 5 oder 6, die ferner aufweist:

eine Hülle (360), die mindestens einen Abschnitt des Verbinders (346) und mindestens einen Abschnitt des Endeffektors umhüllt, wobei die Umhüllung (360) einen ersten Längsschlitz, durch den das erste Merkmal des Endeffektors ausfährt und einfährt, und einen zweiten Längsschlitz aufweist, durch den das zweite Merkmal des Endeffektors ausfährt und einfährt;
ein erstes Kabel (348a), das ein erstes Ende, das mit der Hülle (360) gekoppelt ist, und ein zweites Ende aufweist, das mit dem ersten Merkmal gekoppelt ist; und
ein zweites Kabel (348b), das ein erstes Ende, das mit der Hülle (360) gekoppelt ist, und ein zweites Ende aufweist, das mit dem zweiten Merkmal gekoppelt ist,
wobei eine erste Verschiebung des Endeffektors in der ersten Längsrichtung das erste Merkmal und das zweite Merkmal zueinander hin schwenkt, und wobei eine zweite Verschiebung des Endeffektors in der zweiten Längsrichtung das erste Merkmal und das zweite Merkmal voneinander weg schwenkt.

8. Medizinische Vorrichtung nach Anspruch 2, wobei der Verbinder (446) eine zylindrische Stange aufweist, und wobei mindestens ein Teil der zylindrischen Stange ein mit einem Gewinde versehenes Äußeres (4462) aufweist, und wobei der Endeffektor mit dem mit einem Gewinde versehenen Äußeren in Eingriff steht, so dass (a) die erste Drehung des Schafts in der ersten Richtung bewirkt, dass sich der Endeffektor entlang dem mit einem Gewinde versehenen Äußeren in einer ersten Längsrichtung verschiebt, und (b) die zweite Drehung des Schafts in der zweiten Richtung bewirkt, dass sich der Endeffektor entlang dem mit einem Gewinde versehenen Äußeren in einer zweiten Längsrichtung verschiebt.

9. Medizinische Vorrichtung nach Anspruch 2, wobei der Verbinder (546) ein Zwischenzahnrad (5462) und eine Gewindestange (5464) aufweist, wobei die Gewindestange (5464) (a) einen ersten Gewindeabschnitt (5465a), der an einer Mitte einer ersten Seite des Zwischenzahnrads befestigt ist und sich von dieser erstreckt, und (b) einen zweiten Gewindeabschnitt (5465b) aufweist, der an der Mitte einer zweiten Seite des Zwischenzahnrads befestigt ist und sich von dieser erstreckt,

wobei der drehbare Schaft (544) ein mit einem Gewinde versehenes Äußeres aufweist, das in das Zwischenzahnrad in Eingriff steht,
wobei das erste Merkmal des Endeffektors mit dem ersten Gewindeabschnitt (5465a) der Gewindestange (5464) in Eingriff steht, und wobei das zweite Merkmal des Endeffektors mit dem zweiten Gewindeabschnitt (5465b) der Gewindestange (5464) in Eingriff steht.

10. Medizinische Vorrichtung nach Anspruch 9, wobei der erste Gewindeabschnitt (5465a) in einer ersten Richtung und der zweite Gewindeabschnitt (5465b) in einer zweiten Richtung mit einem Gewinde versehen ist, und wobei die zweite Richtung der ersten Richtung entgegengesetzt ist.

11. Medizinische Vorrichtung nach Anspruch 3, wobei der Verbinder eine erste Seite aufweist, die mit dem drehbaren Schaft (644) gekoppelt ist, und eine zweite Seite, die ein darauf ausgebildetes Spiralgewinde (6466) aufweist, wobei das erste Merkmal des Endeffektors ein erstes Ende aufweist, das mit einer Nut des Spiralgewindes in Eingriff steht, und wobei das zweite Merkmal des Endeffektors ein zweites Ende aufweist, das mit der Nut des Spiralgewindes (6466) in Eingriff steht.

12. Medizinische Vorrichtung nach Anspruch 1, wobei der drehbare Schaft (744, 844) ein mit einem Gewinde versehenes Äußeres aufweist, und wobei das zweite Merkmal des Endeffektors eine proximale Oberfläche aufweist, die eine erste Mehrzahl von Zähnen (7312) aufweist, die mit dem mit einem Gewinde versehenen Äußeren des drehbaren Schafts (744, 844) in Eingriff stehen.

13. Medizinische Vorrichtung nach Anspruch 12, wobei das erste Merkmal des Endeffektors eine proximale Oberfläche aufweist, die eine zweite Mehrzahl von Zähnen (8272) aufweist, die mit dem mit einem Gewinde versehenen Äußeren des drehbaren Schafts (44) in Eingriff stehen, und wobei der drehbare Schaft zwischen der proximalen Oberfläche des ersten Merkmals und der proximalen Oberfläche des zweiten Merkmals angeordnet ist.

14. Medizinische Vorrichtung nach einem der vorhergehenden Ansprüche, die ferner eine Steuerung aufweist, die mit dem Motor (142, 242, 342, 442, 542, 642, 742, 842) in Verbindung steht, wobei die Steuerung konfiguriert ist, eine Richtung der Drehung des Schafts, die durch den Motor (142, 242, 342, 442, 542, 642, 742, 842) vermittelt wird, auszuwählen und/oder zu ändern.

15. Medizinische Vorrichtung nach einem der vorhergehenden Ansprüche, wobei der Endeffektor eine Klammervorrichtung (212) ist, wobei das erste Merkmal einen Amboss (130, 230, 330, 430, 530, 630, 730, 830, 930, 930a) der Klammervorrichtung (212) aufweist, und wobei das zweite Merkmal ein Kassettengehäuse (126, 226, 326, 426, 526, 626, 726, 826, 926) der Klammervorrichtung (212) aufweist.

**Revendications**

1. Dispositif médical, comprenant :

   un effecteur terminal, comprenant une première caractéristique et une deuxième caractéristique, dans lequel la première caractéristique et la deuxième caractéristique sont assemblées l'une à l'autre de manière pivotante ; et
   un moteur (142, 242, 342, 442, 542, 642, 742, 842) couplé à un arbre rotatif (144, 244, 744), dans lequel le moteur (142, 242, 342, 442, 542, 642, 742, 842) est disposé au niveau d'une partie distale du dispositif à proximité de l'effecteur terminal, et dans lequel la partie distale à proximité de l'effecteur terminal est configurée pour s'étendre à l'intérieur du corps d'un sujet pendant une procédure médicale,
   dans lequel l'arbre rotatif (144, 244, 744) met en prise l'effecteur terminal, et dans lequel le moteur (142, 242, 342, 442, 542, 642, 742, 842) est configuré pour faire tourner l'arbre rotatif (144, 244, 744) dans une première direction et une deuxième direction, dans lequel la deuxième direction est opposée à la première direction ;
   dans lequel une première rotation de l'arbre rotatif (144, 244, 744) dans la première direction est configurée pour faire pivoter la première caractéristique et la deuxième caractéristique l'une vers l'autre, et
   dans lequel une deuxième rotation de l'arbre rotatif (144, 244, 744) dans la deuxième direction est configurée pour faire pivoter la première caractéristique et la deuxième caractéristique à l'opposé l'une de l'autre.

2. Dispositif médical selon la revendication 1, comprenant en outre un connecteur (146, 246, 346, 446, 546, 646) couplé à l'arbre rotatif (144, 244, 744), dans lequel le connecteur (146, 246, 346, 446, 546, 646) est configuré pour mettre directement en prise l'effecteur terminal.

3. Dispositif médical selon la revendication 2, comprenant en outre :

   un premier câble (148a), comprenant une première extrémité couplée au connecteur (146) et une deuxième extrémité couplée à la première caractéristique ; et
   un deuxième câble (148b), comprenant une première extrémité couplée au connecteur (146) et une deuxième extrémité couplée à la deuxième caractéristique ;
   dans lequel le connecteur (146) comprend une lumière filetée,
   dans lequel l'arbre rotatif (144) comprend un extérieur fileté, mettant en prise la lumière filetée du connecteur (146), et
   dans lequel la première rotation de l'arbre rotatif (144) dans la première direction fait effectuer une translation au connecteur (146) dans une première direction longitudinale, et dans lequel la deuxième rotation de l'arbre rotatif (144) dans la deuxième direction fait effectuer une translation au connecteur (146) dans une deuxième direction longitudinale.

4. Dispositif médical selon la revendication 3, dans lequel une première translation du connecteur (146) dans la première direction longitudinale fait pivoter la première caractéristique et la deuxième caractéristique l'une vers l'autre, et dans lequel la deuxième translation du connecteur (146) dans la deuxième direction longitudinale amène le premier câble (148a) à tirer sur la première caractéristique et le deuxième câble (148b) à tirer sur la deuxième caractéristique, faisant ainsi pivoter la première caractéristique et la deuxième caractéristique à l'opposé l'une de l'autre.

5. Dispositif médical selon la revendication 2, dans lequel le connecteur (246, 346, 446) comprend une lumière filetée,

   dans lequel l'effecteur terminal comprend en outre un corps proximal par rapport à la première caractéristique et à la deuxième caractéristique, dans lequel le corps comprend un extérieur fileté,
   dans lequel la lumière filetée du connecteur (246, 346, 446) met en prise l'extérieur fileté du corps de l'effecteur terminal, et
   dans lequel la première rotation de l'arbre rotatif (244, 344, 344) dans la première direction fait effectuer une translation à l'effecteur terminal dans une première direction longitudinale, par rapport au connecteur (246, 346, 446), et dans lequel la deuxième rotation de l'arbre rotatif (244, 344, 344) dans la deuxième direction fait effectuer une translation à l'effecteur terminal dans une deuxième direction longitudinale, par rapport au connecteur (244, 344, 344).

6. Dispositif médical selon la revendication 5, dans lequel la première caractéristique comprend un premier filetage et la deuxième caractéristique comprend un deuxième filetage,

dans lequel la lumière filetée du connecteur (346) met en prise le premier filetage de la première caractéristique et le deuxième filetage de la deuxième caractéristique, et

dans lequel une première translation de l'effecteur terminal dans la première direction longitudinale fait pivoter la première caractéristique et la deuxième caractéristique l'une vers l'autre, et dans lequel une deuxième translation de l'effecteur terminal dans la deuxième direction longitudinale fait pivoter la première caractéristique et la deuxième caractéristique à l'opposé l'une de l'autre.

7. Dispositif médical selon les revendications 5 ou 6, comprenant en outre :

une gaine (360), gainant au moins une partie du connecteur (346) et au moins une partie de l'effecteur terminal, dans lequel la gaine (360) comprend une première fente longitudinale à travers laquelle la première caractéristique de l'effecteur terminal s'étend et se rétracte, et une deuxième fente longitudinale à travers laquelle la deuxième caractéristique de l'effecteur terminal s'étend et se rétracte ;

un premier câble (348a) comprenant une première extrémité couplée à la gaine (360) et une deuxième extrémité couplée à la première caractéristique ; et

un deuxième câble (348b) comprenant une première extrémité couplée à la gaine (360) et une deuxième extrémité couplée à la deuxième caractéristique ;

dans lequel une première translation de l'effecteur terminal dans la première direction longitudinale fait pivoter la première caractéristique et la deuxième caractéristique l'une vers l'autre, et dans lequel une deuxième translation de l'effecteur terminal dans la deuxième direction longitudinale fait pivoter la première caractéristique et la deuxième caractéristique à l'opposé l'une de l'autre.

8. Dispositif médical selon la revendication 2, dans lequel le connecteur (446) comprend une barre cylindrique, et dans lequel au moins une partie de la barre cylindrique comprend un extérieur fileté (4462), et dans lequel l'effecteur terminal met en prise l'extérieur fileté, de sorte que (a) la première rotation de l'arbre dans la première direction amène l'effecteur terminal à effectuer une translation longitudinale le long de l'extérieur fileté dans une première direction longitudinale, et (b) la deuxième rotation de l'arbre dans la deuxième direction amène l'effecteur terminal à effectuer une translation longitudinale le long de l'extérieur fileté dans une deuxième direction longitudinale.

9. Dispositif médical selon la revendication 2, dans lequel le connecteur (546) comprend un engrenage intermédiaire (5462) et une barre filetée (5464), dans lequel la barre filetée (5464) comprend (a) une première partie filetée (5465a) fixée à et s'étendant depuis un centre d'un premier côté de l'engrenage intermédiaire, et (b) une deuxième partie filetée (5465b) fixée à et s'étendant depuis le centre d'un deuxième côté de l'engrenage intermédiaire,

dans lequel l'arbre rotatif (544) comprend un extérieur fileté mettant en prise l'engrenage intermédiaire, dans lequel la première caractéristique de l'effecteur terminal met en prise la première partie filetée (5465a) de la barre filetée (5464), et dans lequel la deuxième caractéristique de l'effecteur terminal met en prise la deuxième partie filetée (5465b) de la barre filetée (5464).

10. Dispositif médical selon la revendication 9, dans lequel la première partie filetée (5465a) est filetée dans une première direction et la deuxième partie filetée (5465b) est filetée dans une deuxième direction, et dans lequel la deuxième direction est opposée à la première direction.

11. Dispositif médical selon la revendication 3, dans lequel le connecteur comprend un premier côté couplé à l'arbre rotatif (644), et un deuxième côté incluant un filetage en spirale (6466) formé sur ce dernier, dans lequel la première caractéristique de l'effecteur terminal comprend une première extrémité mettant en prise une rainure du filetage en spirale, et dans lequel la deuxième caractéristique de l'effecteur terminal comprend une deuxième extrémité mettant en prise la rainure du filetage en spirale (6466).

12. Dispositif médical selon la revendication 1, dans lequel l'arbre rotatif (744, 844) comprend un extérieur fileté, et

dans lequel la deuxième caractéristique de l'effecteur terminal comprend une surface proximale comprenant une première pluralité de dents (7312) mettant en prise l'extérieur fileté de l'arbre rotatif (744, 844).

13. Dispositif médical selon la revendication 12, dans lequel la première caractéristique de l'effecteur terminal comprend une surface proximale comprenant une deuxième pluralité de dents (8272) mettant en prise l'extérieur fileté de l'arbre rotatif (44), et dans lequel l'arbre rotatif est positionné entre la surface proximale de la première caractéristique et la surface proximale de la deuxième caractéristique.

14. Dispositif médical selon l'une quelconque des revendications précédentes, comprenant en outre un organe de commande en communication avec le moteur (142, 242, 342, 442, 542, 642, 742, 842), dans lequel l'organe de commande est configuré pour sélectionner et/ou modifier une direction de la rotation de l'arbre communiquée par le moteur (142, 242, 342, 442, 542, 642, 742, 842).

15. Dispositif médical selon l'une quelconque des revendications précédentes, dans lequel l'effecteur terminal est une agrafeuse (212), dans lequel la première caractéristique comprend une enclume (130, 230, 330, 430, 530, 630, 730, 830, 930, 930a) de l'agrafeuse (212), et dans lequel la deuxième caractéristique comprend un boîtier de cartouche (126, 226, 326, 426, 526, 626, 726, 826, 926) de l'agrafeuse (212).

**FIG. 1A**

**FIG. 1B**

**FIG. 2**

**FIG. 3**

*FIG. 4A*

*FIG. 4B*

FIG. 5A

FIG. 5B

FIG. 5C

**FIG. 6A**

**FIG. 6B**

**FIG. 7A**

**FIG. 7B**

**FIG. 7C**

**FIG. 7D**

**FIG. 8**

**FIG. 9**

**FIG. 10A**

**FIG. 10B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019125357 A1